# EUROPEAN PATENT APPLICATION

(11) **EP 3 184 121 A2**
(43) Date of publication of application: **28.06.2017**
(21) Application number: 16180137.8
(22) Date of filing: 23.07.2013
(51) Int. Cl.: A61K 39/395, A61P 31/12, A61P 35/00, A61K 45/06, A61K 38/17, A61K 9/127, A61K 31/00, A61P 37/06, C12Q 1/70, C07K 16/24, C12Q 1/02, A61K 31/444

(54) **LIPID MEMBRANES WITH EXPOSED PHOSPHATIDYLSERINE AS TAM LIGANDS, USE FOR TREATING AUTOIMMUNE DISEASES**

(30) Priority: 25.07.2012 US 201261675731 P
(62) Divisional of application: 13823490.1
(71) Applicant: Salk Institute For Biological Studies, La Jolla, CA 92037-1099 (US); Celldex Therapeutics, Inc., Hampton, NJ 08827 (US)
(72) Inventor: LEMKE, Greg, E., La Jolla, CA California 92037 (US); FRITZ, Lawrence, C., Rancho Santa Fe, CA California 92067 (US); VOLLRATH, Benedikt, San Diego, CA California 92107 (US); ROTHLIN, Carla, V., New Haven, CT Connecticut 06510 (US)
(74) Representative: Jones Day

(57) **Abstract**

The invention provides a TAM receptor agonist for use in a method of treating an autoimmune disease in a subject, wherein the TAM receptor agonist comprises a phosphatidyl serine (PtdSer) -containing lipid bilayer membrane with Gas6 and/or Protein S bound to the membrane, wherein signaling from one or more TAM receptors is activated. The invention further provides a composition comprising a PtdSer-containing membrane having a Gas6 and/or Protein S protein bound to said membrane. Further provided is a method for classifying a virus as susceptible to anti-TAM therapy and a method of identifying an agent that blocks virus infectivity.

## Description

### CROSS REFERENCE TO RELATED APPLICATION

This application claims priority to U.S. Provisional Application No. 61/675,731 filed July 25, 2012, herein incorporated by reference.

### FIELD

The present disclosure relates to methods for modulating the interaction between a TAM ligand and a lipid membrane containing phosphatidyl serine (PtdSer). For example, increasing this interaction can be used to treat an autoimmune disorder, while decreasing this interaction can be used to treat a viral infection by a virus having PtdSer on its envelope.

### ACKNOWLEDGMENT OF GOVERNMENT SUPPORT

This invention was made with government support under PO1 AI090935; UO1 AI074539; RO1 AI077058; RO1 R01AI089824; and P30 CA014195-38 awarded by the National Institutes of Health. The government has certain rights in the invention.

### BACKGROUND

The TAM receptor tyrosine kinases (RTKs) Tyro3, Axl, and Mer (*1*, *2*), and their cognate ligands Protein S and Gas6 (*3*), promote the phagocytic clearance of apoptotic cells in the mature immune, nervous and reproductive systems (*2*, *4*). They simultaneously drive a critical negative feedback loop that down-regulates host innate immune responses mediated by Toll-like receptor (TLR) and type I interferon signaling pathways (*2*, *5-7*). TAM receptor-ligand interactions have also been implicated in promoting the cellular entry of enveloped viruses: ectopic introduction of one or more TAM receptors into infection-resistant cell lines has been found to potentiate infection by both filoviruses and lentiviruses (*8-13*). In addition to binding to TAM receptors through a carboxy-terminal domain, the TAM ligands Protein S and Gas6 both contain a glutamic acid-rich Gla domain at their amino terminus, which binds to phosphatidylserine (PtdSer) exposed on the surfaces of apoptotic cells and membrane-enveloped virions (*2*, *14*).

### SUMMARY

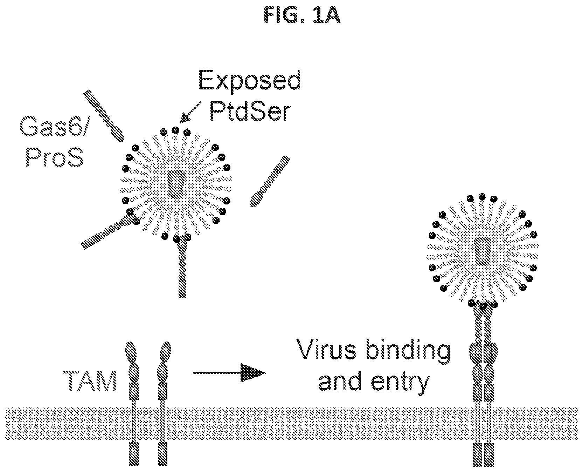

Enveloped viruses contain high levels of PtdSer on their surface and bind TAM ligands Protein S and Gas6 through the PtdSer-Gla domain interaction. The function of TAM receptor-ligand interactions during enveloped virus infection has been thought to be restricted to the facilitation of virus binding to the target cell, thereby facilitating viral infection (*8-13*) (FIG. 1A). Surprisingly, however, the interaction between membrane PtdSer and the gla domain on the TAM ligands changes the ligand properties of the ligand with regard to TAM signaling. It is shown herein that membrane-bound TAM ligands are significantly more potent in activating TAM receptor signaling than free ligands, indicating that PtdSer-binding of TAM ligands through their gla-domain changes the ligand's impact on TAM signal transduction. Methods and compositions are provided by which the interaction between TAM ligands and phosphatidylserine (PtdSer) in a membrane can be modulated, to increase or decrease TAM receptor activity.

The present disclosure provides methods of treating an autoimmune disorder in a subject. In some examples, such a method includes administering to a subject having an autoimmune disorder a therapeutically effective amount of a TAM receptor agonist comprising a phosphatidyl serine (PtdSer) -containing lipid bilayer membrane (such as a liposome or a nanoparticle) with Gas6 and/or Protein S bound to the membrane, thereby activating signaling from one or more TAM receptors and treating the autoimmune disease. Exemplary autoimmune disorders include, but are not limited to: rheumatoid arthritis, Hashimoto's thyroiditis, pernicious anemia, inflammatory bowel disease, psoriasis, renal, pulmonary, and hepatic fibroses, Addison's disease, type I diabetes, systemic lupus erythematosus, dermatomyositis, Sjogren's syndrome, multiple sclerosis, myasthenia gravis, Reiter's syndrome, and Grave's disease.

Disclosed herein are compositions that include a PtdSer-containing membrane having a Gas6 and/or Protein S protein bound to said membrane. In some examples, the PtdSer containing membrane comprises a liposome or a nanoparticle. Such compositions can be used to treat an autoimmune disease.

Methods of treating a subject with a pathological condition characterized by overactivation of TAM signaling and/or reduction in Type I IFN response are provided. An example of such a pathological condition is an infection by an enveloped virus. In one example, such a method includes administering a therapeutically effective amount of an agent that can disrupt the interaction between Gas6 and/or Protein S with PtdSer-containing lipid bilayer membranes, thereby decreasing TAM receptor activity.

Also provided are methods for classifying a virus as susceptible to anti-TAM therapy. In one example, anti-TAM therapy is an inhibitor of TAM signaling, such as a small molecule kinase inhibitor blocking the RTK functionality of TAM. Such methods can include determining whether the virus has PtdSer on its envelope surface; and/or determining whether the virus induces TAM signaling when in contact with a TAM ligand. In some examples, such a method includes determining an amount (such as a qualitative or quantitative value) of PtdSer on the virus envelope surface. The resulting value can be compared with a value for a virus with a known amount of PtdSer on the envelope surface and/or a virus known to induce TAM signaling, thereby classifying the virus as susceptible or resistant to anti-TAM therapy.

Methods of identifying an agent that blocks virus infectivity are also provided. In some examples, such methods include contacting one or more test agents with a virus and an immune cell (such as a dendritic cell or macrophage) and determining TAM receptor activation in the presence and absence of the test agent(s). The presence of decreased TAM receptor activation indicates the test agent(s) can block virus infectivity.

The foregoing and other objects and features of the disclosure will become more apparent from the following detailed description, which proceeds with reference to the accompanying figures.

### BRIEF DESCRIPTION OF THE DRAWINGS

**FIGS. 1A-1B** **show loss-of-function studies to evaluate the impact of specific TAM receptors on virus infection. (A)** Current model of TAM receptor-ligand facilitation of enveloped virus entry. (**B**) BMDCs obtained from wild-type or specific TAM receptor knockout mice were challenged with the HIV-1 derived virus pseudotyped with the indicated viral glycoproteins and the subsequent levels of HIV-1 DNA were measured. Levels shown were normalized to those seen with WT-BMDCs (100%; dashed line). KO, DKO, and TKO represent single-, double-, and triple-TAM receptor knockouts, respectively. *, **, and *** represent p-values of <0.05, <0.01, and <0.001, respectively. Error bars represent SEM of three independent samples.

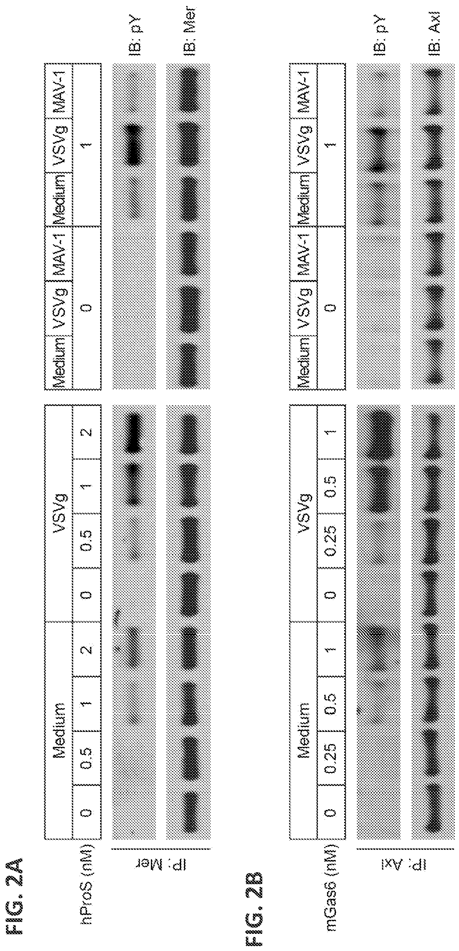

**FIGS. 2A-2B** **show that enveloped virus potentiates ligand-dependent TAM receptor activation during virus entry. (A) Left** WT-BMDCs were incubated with increasing concentrations (0.5-2 nM) of purified human Protein S (hProS), in either Protein S-depleted cell culture medium alone (Medium), or together with enveloped VSVg-pseudotyped HIV-1-derived virus that was produced in Protein S-depleted medium (VSVg). Cell lysates were prepared for immunoblot analysis 5 minutes after virus/hProS challenge. Mer was specifically immunoprecipitated from these samples, subjected to SDS-PAGE, and then immunoblotted with a phosphotyrosine-specific antibody (IB: pY). Immunoblot analysis was used to confirm equal amounts of Mer in each sample (IB: Mer). **Right**, Virus potentiation of Mer auto-phosphorylation in the presence of 1 nM hProS for 5 minutes is seen for the enveloped VSVg pseudotyped virus, but not with mouse adenovirus type-1 (WT-MAV-1), a control non-enveloped virus. (**B**) **Left**, The same experiments as in panel A (left), except that WT-BMDCs were incubated with increasing concentrations (0.25-1 nM) of recombinant mouse Gas6 (mGas6) and the tyrosine auto-phosphorylation status of Ax1 was monitored (IB: pY). **Right** Virus potentiation of Axl auto-phosphorylation in the presence of 1 nM mGas6 was seen only with the enveloped VSVg pseudotyped virus, and not with the non-enveloped MAV-1 control virus.

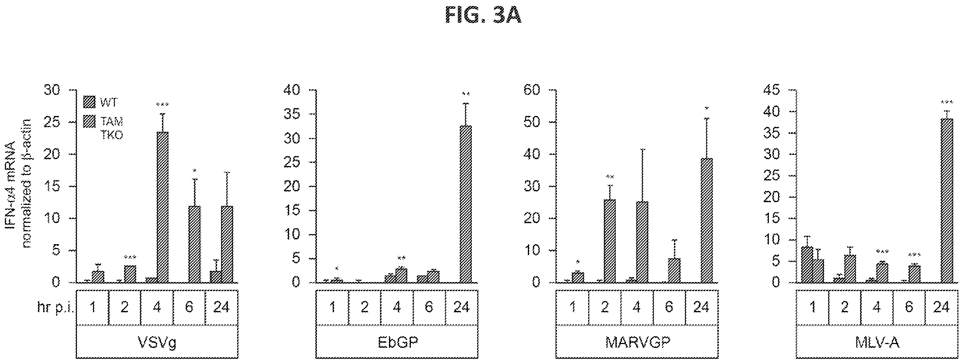

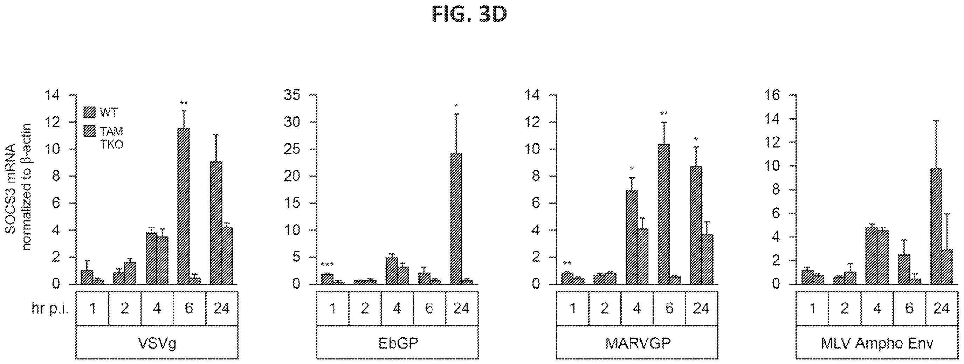

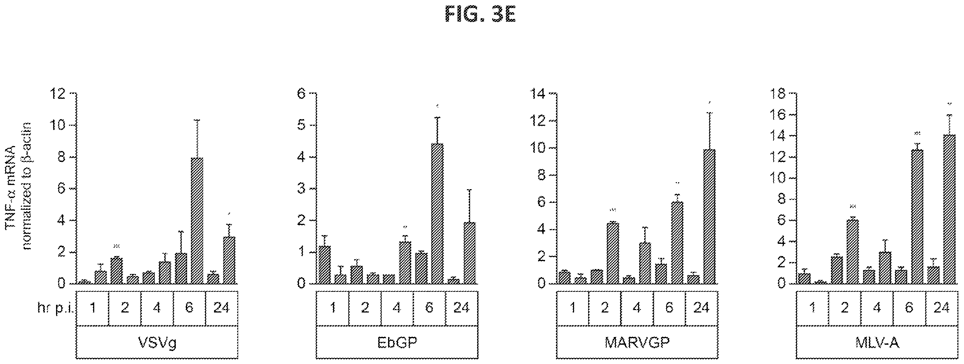

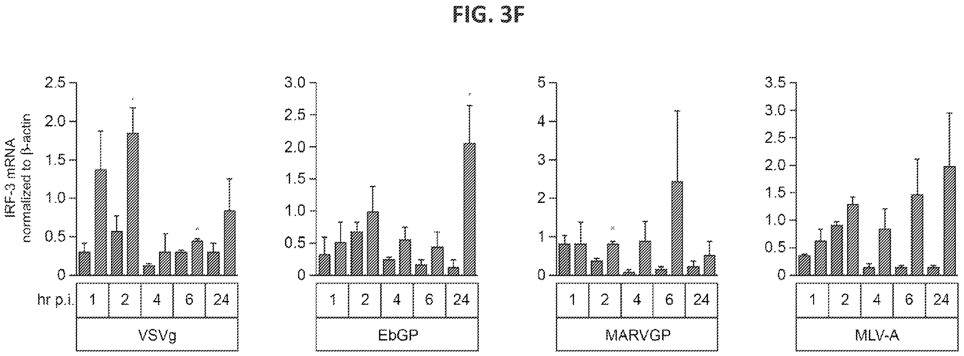

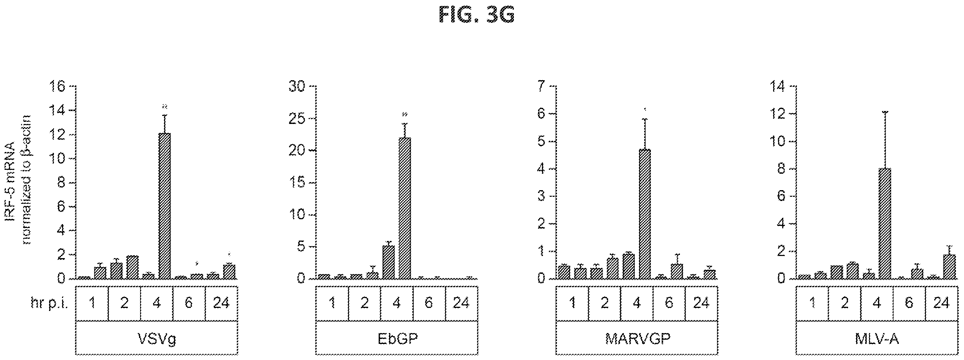

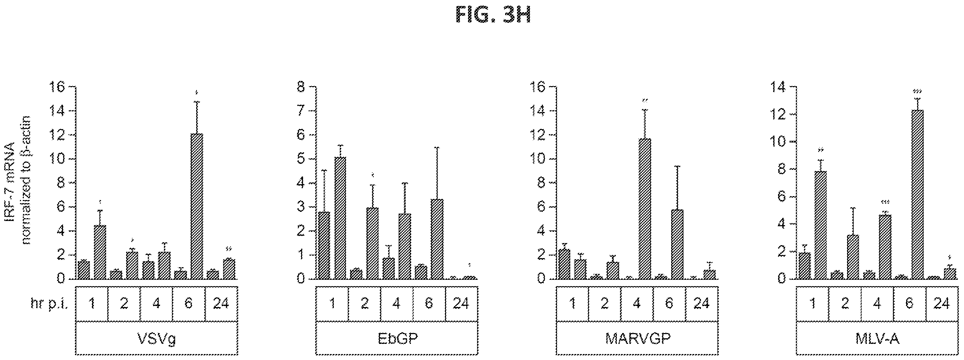

**FIGS. 3A-H** **show that enveloped virus infection abrogates the cellular antiviral response in a TAM-dependent manner.** (**A**-**C**), WT-BMDCs (left bar for each group) or TAM TKO-BMDCs (right bar for each group) were challenged with the indicated pseudotyped viruses. The mRNA expression levels of IFN-α4 (**A**), IFN-β (**B**), and SOCS-1 (**C**) mRNAs were measured by quantitative RT-PCR amplification at the indicated time points after virus addition. The levels of the different gene-specific mRNAs were normalized to that of the control β-actin gene. *, **, and *** represent p-values of <0.05, <0.01, and <0.001, respectively. Results comparable to those for IFNα4 and IFNβ mRNAs were seen for measurements of TNF- α, IRF-3, IRF-5, and IRF-7 mRNAs; and results comparable to those for SOCS1 mRNA were seen for SOCS3 mRNA (FIGS. 3D-H). Error bars represent SEM of three independent samples.

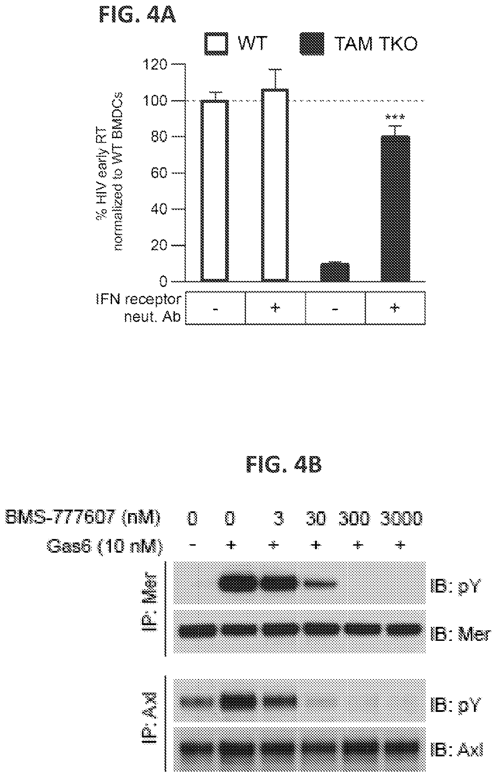

**FIGS. 4A-4D** **show the major effect of TAM receptor-ligand interactions on virus infection in BMDCs is through inhibition of the cellular antiviral response.** (**A**) WT-BMDCs and TAM TKO-BMDCs were incubated with the VSVg-pseudotyped virus either in the absence (-) or presence (+) of 100mg each of neutralizing anti-mouse interferon alpha and beta antibodies and the resultant levels of HIV-1 DNA were measured at 24 h post-infection. The results shown were normalized to the level of viral DNA seen with WT-BMDCs incubated with no antibody (100%; blue dashed line). Error bars represent SEM of three independent samples. (**B**) WT-Bone marrow derived macrophages (BMDMs) were preincubated with or without the BMS-777607 compound and then stimulated with Gas6 for 10 min. Receptor activation was monitored using the same immunoprecipitation-immunoblotting protocol used in Fig. 2 that employed a phosphotyrosine-specific antibody (pY). Immunoblot analysis was used to confirm equal amounts of Mer, Axl and Tyro3 in each sample. (**C**) WT-BMDCs and TAM TKO-BMDCs were incubated with the EbGP-pseudotyped virus either in the absence (-) or presence (+) of 300 nM BMS-777607 and the subsequent levels of HIV-1 viral DNA were measured at 24 h post-infection. The results shown were normalized those seen with WT-BMDCs (100%, dashed line). ** represents p-value <0.01. Error bars represent SEM of three independent samples. (**D**) Model showing that the major effect of TAM receptor-ligand interactions on enveloped virus infection of dendritic cells is at the level of inhibiting the cellular innate immune response.

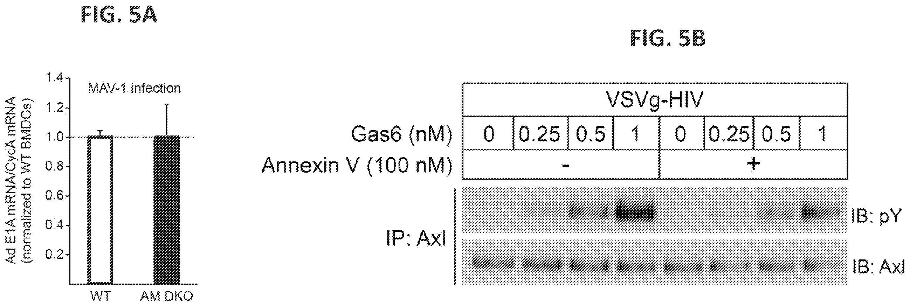

**FIGS 5** **A and 5B show that a PtdSer-containing membrane envelope is necessary for virus activation of TAM signaling.** (**A**) MAV-1 infects WT and Axl-/-Mer-/- (AM DKO) cells with equal efficiency. MAV-1 was grown on mouse 3T6 cells, concentrated from supernatant using PEG precipitation, and purified on a CsCl gradient. Purified virus was dialyzed in 10% glycerol, 10mM Tris pH7.5, 150mM NaCl, and 1mM MgCl₂, and titered by plaque assay on 3T6 cells. BMDCs were infected with MAV-1 at MOI of 1. Cells were lysed 24 h postinfection, and expression of early adenovirus gene E1A was assayed by qPCR and normalized to endogenous Cyclophilin A. (**B**) Annexin V inhibition of VSVg-HIV potentiation of Gas6-driven Axl activation in BMDCs. Experiments were performed and Axl activation (autophosphorylation) assayed as described for FIG. 2, but in the absence (-) or presence (+) of 100 nM of the PtdSer-binding protein Annexin V. This concentration of Annexin V is approximately 7-fold lower than that used previously by Meertens *et al.* (2012) to achieve a ∼60% reduction in DENV infection of Axl-expressing HEK293 cells.

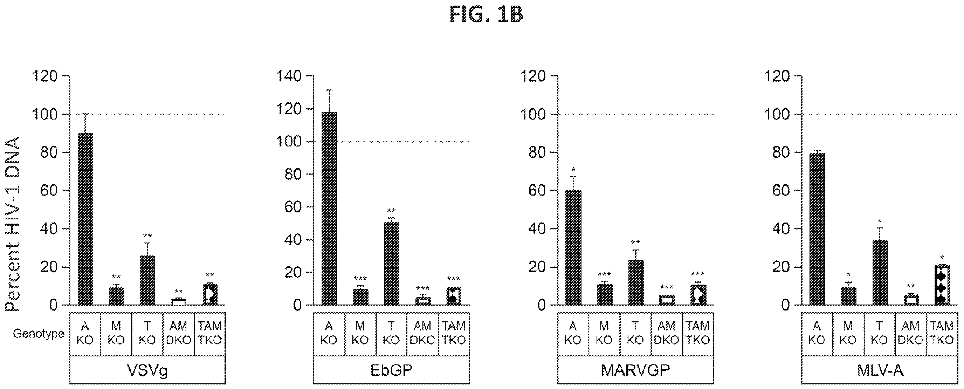

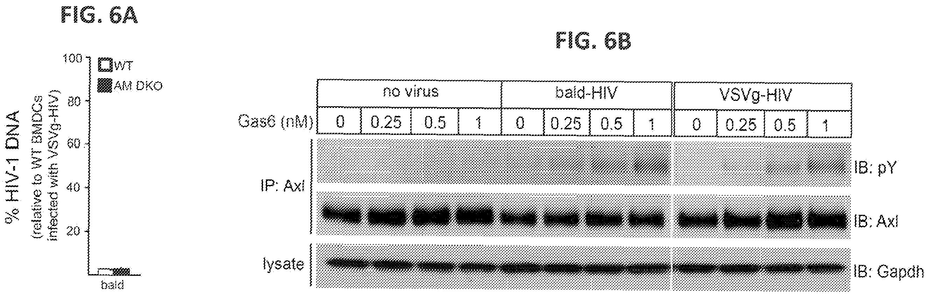

**FIGS 6** **A and 6B show that Retrovirus potentiation of TAM signaling is independent of any viral glycoprotein.** (**A**) Infection and assay of mouse BMDCs, performed as described for the experiments of FIG. 1B, demonstrate that a 'bald' HIV-1-derived retrovirus lacking any exogenous viral glycoprotein is not infectious for either WT (left bar) or AM DKO BMDCs (right bar). (**B**) This non-infectious bald retrovirus, which still carries a PtdSer-containing membrane envelope, potentiates Gas6-driven Axl activation as well as a VSVg-pseudotyped retrovirus.

### SEQUENCE LISTING

The nucleic acid sequences listed in the accompanying sequence listing are shown using standard letter abbreviations for nucleotide bases, as defined in 37 C.F.R. 1.822. Only one strand of each nucleic acid sequence is shown, but the complementary strand is understood as included by any reference to the displayed strand. In the accompanying sequence listing:
**SEQ ID NOS: 1**-**22** are the nucleic acid sequences of probes and primers used in the experiments herein.

### DETAILED DESCRIPTION

Unless otherwise explained, all technical and scientific terms used herein have the same meaning as commonly understood by one of ordinary skill in the art to which this disclosure belongs. The singular terms "a," "an," and "the" include plural referents unless context clearly indicates otherwise. "Comprising" means "including." "Comprising A or B" means "including A," "including B," or "including A and B." It is further to be understood that all base sizes or amino acid sizes and all molecular weight or molecular mass values given for nucleic acids or peptides are approximate, and are provided for description.

All publications, patent applications, patents, and other references mentioned herein are incorporated by reference in their entirety for all purposes. All sequences associated with the GenBank® Accession numbers mentioned herein are incorporated by reference in their entirety as were present on July 25, 2012. Although exemplary GenBank® numbers are listed herein, the disclosure is not limited to the use of these sequences.

Suitable methods and materials for the practice or testing of the disclosure are described below. However, the provided materials, methods, and examples are illustrative only and are not intended to be limiting. Accordingly, except as otherwise noted, the methods and techniques of the present disclosure can be performed according to methods and materials similar or equivalent to those described and/or according to conventional methods well known in the art and as described in various general and more specific references that are cited and discussed throughout the present specification.

In order to facilitate review of the various embodiments of the disclosure, the following explanations of specific terms are provided:
**Administration:** Introduction of an agent into a subject, such as an agent that modulates an interaction between a TAM ligand and phosphatidyl serine (PtdSer). Includes oral, rectal, vaginal, transdermal, nasal, and parenteral administration. Generally, parenteral formulations are those that are administered through any possible mode except ingestion. This term also refers to injections, whether administered intravenously, intrathecally, intramuscularly, intraperitoneally, intra-articularly, or subcutaneously, and various surface applications including intranasal, inhalational, intradermal, and topical application, for instance.

**Anti-viral agent:** A substance (such as a chemical compound, protein, antibody, antisense or RNAi oligonucleotide, or other molecule) for use in treating a viral infection in a subject. In some examples, an agent that disrupts an interaction between a TAM ligand and PtdSer present in a membrane is used in combination with other anti-viral compounds. Anti-viral agents include, but are not limited to, zidovudine, didanosine, zalcitabine, stavudine, lamivudine, abacavir, tenofovir, nevirapine, delavirdine, efavirenz, saquinavir, ritonavir, indinavir, nelfinavir, saquinavir, amprenavir, and lopinavir.

**Autoimmune disorder:** A disease in which the immune system produces an immune response (for instance, a B cell or a T cell response) against an endogenous antigen, with consequent injury to tissues. Exemplary autoimmune disorders include, but are not limited to: amyotriphic lateral sclerosis, celiac disease, rheumatoid arthritis, Hashimoto's thyroiditis, pernicious anemia, inflammatory bowel disease (Crohn's disease and ulcerative colitis), Cushing's syndrome, Guillain-Barre syndrome, transverse myelitis, psoriasis, renal, pulmonary, and hepatic fibroses, Addison's disease, type I diabetes, systemic lupus erythematosus, dermatomyositis, Sjogren's syndrome, multiple sclerosis, myasthenia gravis, Reiter's syndrome, and Grave's disease, among others.

**Contact:** To bring one agent into close proximity to another agent, thereby permitting the agents to interact. For example, a test agent can be added to cells and viruses in culture, thereby allowing the agent to interact with PtdSer in the viral membrane and reduce or inhibit TAM receptor activation by the cell. In another example, cells in a mammal are contacted with an agent that either increases or disrupts an interaction between a TAM ligand and PtdSer present in a membrane by administration of the agent to the mammal.

**Detect:** To determine if an agent (*e.g.,* PtdSer) or activity (*e.g.,* TAM receptor activity) is present or absent. In some examples this can further include quantification. Detection can be in bulk, so that a macroscopic number of molecules can be observed simultaneously. Detection can also include identification of signals from single molecules using microscopy and such techniques as total internal reflection to reduce background noise.

**Enveloped virus:** A virus having a viral envelope covering its protein capsid. Envelopes are typically derived from portions of the host cell membranes (phospholipids and proteins), but include some viral glycoproteins. Prior to infection, the viral envelope fuses with the host cell membrane, allowing the capsid and viral genome to enter and infect the host cell. Glycoproteins on the surface of the envelope serve to identify and bind to receptor components on the host's membrane.

Examples of enveloped viruses include, but are not limited to: influenza, Semliki Forest Virus (SFV), filoviruses (Ebola virus and Marburg virus), retroviruses (*e.g*., human immunodeficiency virus (HIV), simian immunodeficiency virus (SIV) or feline immunodeficiency virus (FIV)), rabies, Herpes simplex viruses (HSV), cytomegalovirus (CMV), Epstein Barr virus, murine leukemia virus (MLV), hepatitis C virus (HCV), hepatitis B virus (HBV), human papillomavirus (HPV), coxsackie viruses, rhinoviruses, yellow fever virus, West Nile virus, and vesicular stomatitis virus (VSV).

**Filoviruses:** A family of viruses that belong to the order *Mononegavirales.* Filoviruses are single stranded negative-sense RNA viruses that target primates. There are two genera: the Ebola virus and Marburg virus. These viruses cause viral hemorrhagic fevers, characterized by bleeding and coagulation abnormalities, often leading to death.

**IC₅₀:** A measure of concentration used in pharmacology. IC₅₀, or the half maximal inhibitory concentration, represents the concentration of an inhibitor that is required for 50% inhibition of its target (for instance, a TAM receptor or a virus). Generally, an IC₅₀ value is a measure of how much of a particular composition (*e.g*., an agent that disrupts an interaction between a TAM ligand and PtdSer present in a membrane) is needed to inhibit some biological process (*e.g.,* a viral infection) by 50%. IC₅₀ is commonly used as a measure of drug affinity, and represents the concentration of a composition that is required to obtain 50% of the maximum effect *in vivo.*

**Immune response:** A response of a cell of the immune system, such as a B cell or T cell or macrophage, to a stimulus (*e.g*., infection by a virus). A "parameter of an immune response" is any particular measurable aspect of an immune response, including, but not limited to, cytokine secretion (IL-6, IL-10, IFN-α, IFN-β etc.), immunoglobulin production, dendritic cell maturation, and proliferation of a cell of the immune system. "Enhancing an immune response" includes the use of any composition or method that results in an increase in any of these parameters. One of skill in the art can readily determine an increase in any one of these parameters using known laboratory assays. In one specific non-limiting example, an ELISA is used to detect cytokine (*e.g*., IFN-β) secretion. A "substantial" increase in a parameter of the immune response is a significant increase in this parameter (*e.g*., in the presence of a TAM receptor inhibitor) as compared to a control (*e.g.,* in the absence of a TAM receptor inhibitor). Specific, non-limiting examples of a substantial increase are at least about a 10% increase, at least about a 20% increase, at least about a 40% increase, at least about a 50% increase, at least about a 75% increase, at least about a 90% increase, at least about a 100% increase, at least a 3 fold increase, at least about a 10 fold increase, at least about a 20 fold increase. "Reducing an immune response" includes the use of any composition or method that results in a decrease in any of these parameters. One of skill in the art can readily determine a decrease in any one of these parameters using known laboratory assays. In one specific non-limiting example, an ELISA is used to detect cytokine (e*.g.,* IFN-β) secretion. A "substantial" decrease in a parameter of the immune response is a significant decrease in this parameter (*e.g.,* in the presence of a TAM receptor agonist) as compared to a control (*e.g.,* in the absence of a TAM receptor agonist). Specific, non-limiting examples of a substantial decrease are at least about a 10% decrease, at least about a 20% decrease, at least about a 40% decrease, at least about a 50% decrease, at least about a 75% decrease, at least about a 90% decrease, and at least about a 99% decrease.

One of skill in the art can readily identify a significant increase or decrease using known statistical methods. One, specific, non-limiting example of a statistical test used to assess a substantial increase is the use of a Z test to compare the percent of samples that respond to an activated TAM receptor (*e.g.,* TAM receptor inhibitor absent) as compared to the percent of samples that respond to an inactivated TAM receptor (*e.g.,* a TAM receptor inhibitor present). One, specific, non-limiting example of a statistical test used to assess a substantial decrease is the use of a Z test to compare the percent of samples that respond to a deactivated TAM receptor (*e.g.,* TAM receptor agonist absent) as compared to the percent of samples that respond to an activated TAM receptor (*e.g.,* a TAM receptor agonist present). A non-parametric ANOVA can be used to compare differences in the magnitude of the response induced in the absence of a TAM receptor inhibitor as compared to the percent of samples that respond in the presence of a TAM receptor inhibitor. In this example, *p* ≤ 0.05 is significant, and indicates a substantial increase in the parameter of the immune response. One of skill in the art can readily identify other statistical assays of use.

**Interferon (IFN) type I**: Interferons (IFNs) are cytokines produced by the immune cells of vertebrates in response to challenges by viruses (*e.g*., rhinovirus, influenza virus, HIV) as well as some parasites (*e.g., Leishmania*) and bacteria (*e.g., Listeria*)*.* Type I IFNs bind to the cell surface receptor complex known as the IFN-α receptor, and exhibit pleiotropic effects on a wide variety of cell types, including antiviral activity and antibacterial, antiprozoal, immunomodulatory, and cell growth regulatory functions. For example, IFNs can inhibit viral replication within host cells, activate natural killer cells and macrophages, increase antigen presentation to lymphocytes, and induce the resistance of host cells to viral infection. Exemplary type I IFNs include the acid-stable interferons IFN-alpha (IFNα) and IFN-beta (IFN-β), as well as IFN-delta, IFN-omega, IFN-tau, and IFN-kappa. IFN-α and IFN-β are secreted by many cell types including lymphocytes (NK cells, B-cells and T-cells), macrophages, fibroblasts, endothelial cells, osteoblasts and others.

**Interferon alpha (IFN-α):** A type I interferon glycoprotein that is involved in the regulation of humoral immune responses and immune responses against viral infections. IFN-α is produced by leukocytes and other cells and stimulates macrophages in response to stimulation by live or inactivated virus and other agents and has antiviral activity.

There are at least 23 different IFN-alpha genes. They have a length of 1-2 kb and are clustered on human chromosome 9p22. Exemplary IFN-α sequences are known in the art, and are publicly available on GenBank or other databases, such as Genbank Accession Nos. AAA52716.1; NP_076918.1; NP_000596.2 (human proteins) and AAA37886.1; NP_996754.1 (mouse proteins) (sequences of which are herein incorporated by reference for the sequence available on July 25, 2012).

Methods of detecting IFN-α production by a cell are known, and include real time quantitative PCR and ELISA.

**Interferon beta (IFN-β):** A type I interferon glycoprotein that is involved in the regulation of humoral immune responses and immune responses against viral and other pathogenic infections. IFN-β is produced by fibroblasts and other cells in response to stimulation by live or inactivated virus or by double-stranded RNA, and has antiviral, antiproliferative, and immunomodulating activity.

The human gene encoding IFN-β maps to chromosome 9p22 in the vicinity of the IFN-alpha gene cluster. Exemplary IFN-β sequences are known in the art, and are publicly available on GenBank or other databases, such as Genbank Accession Nos. AAC41702.1; NP_002167; CAH70160.1 (human proteins) and AAI19396.1; AAI19398.1 (mouse proteins) (sequences of which are herein incorporated by reference for the sequence available on July 25, 2012).

Methods of detecting IFN-β production by a cell are known, and include real time quantitative PCR and ELISA.

**Interferon response factor (IRF):** Transcription factors that regulate interferon (*e.g*., type I IFN) transcription. This family of proteins has diverse roles, including virus-mediated activation of interferon, and modulation of cell growth, differentiation, apoptosis, and immune system activity. Members of the IRF family are generally characterized by a conserved N-terminal DNA-binding domain containing tryptophan (W) repeats. Examples include interferon regulatory factor 3 (IRF3; OMIM: 603734), a transcription factor critical to the initiation of the antiviral response, IRF5 (OMIM: 607218), a mediator of toll-like receptor (TLR)7 signaling, and IRF7 (OMIM: 605047) which participates in the transcriptional activation of virus-inducible cellular genes. Exemplary IRF sequences are known in the art, and are publicly available on GenBank or other databases, such as Genbank Accession Nos. NP_001562 (protein) and NM_001571 (nucleic acid) (IRF3); NP_002191 and NM_002200 (nucleic acid) (IRF5); and NP_001563 (protein) and M_001572 (nucleic acid) (IRF7) (sequences of which are herein incorporated by reference for the sequence available on July 25, 2012).

**Isolated:** An "isolated" biological component (such as a nucleic acid molecule, peptide, or cell) has been purified away from other biological components in a mixed sample (such as a cell extract). For example, an "isolated" peptide or nucleic acid molecule is a peptide or nucleic acid molecule that has been separated from the other components of a cell in which the peptide or nucleic acid molecule was present (such as an expression host cell for a recombinant peptide or nucleic acid molecule). In one example, an "isolated" composition that includes a PtdSer-containing membrane having a Gas6 and/or Protein S protein bound to said membrane is one not found in nature.

**Pharmaceutically acceptable carriers:** The pharmaceutically acceptable carriers useful in this disclosure are conventional. Remington's Pharmaceutical Sciences, by E. W. Martin, Mack Publishing Co., Easton, PA, 15th Edition (1975), describes compositions and formulations suitable for pharmaceutical delivery of the therapeutic agents provided herein, such as a TAM receptor agonist or antagonist.

In general, the nature of the carrier will depend on the particular mode of administration being employed. For instance, parenteral formulations usually include injectable fluids that include pharmaceutically and physiologically acceptable fluids such as water, physiological saline, balanced salt solutions, aqueous dextrose, glycerol or the like as a vehicle. For solid compositions (*e.g*., powder, pill, tablet, or capsule forms), conventional non-toxic solid carriers can include, for example, pharmaceutical grades of mannitol, lactose, starch, or magnesium stearate. In addition to biologically-neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

**Subject:** Living multi-cellular vertebrate organisms, a category that includes both human and non-human mammals. The methods and compositions disclosed herein have equal applications in medical and veterinary settings. Therefore, the general term "subject" is understood to include all animals, including, but not limited to, humans or veterinary subjects, such as other primates, dogs, cats, horses, and cows.

**TAM receptor:** The TAM family (Tyro3, Axl, and Mer) was first identified as a distinct receptor protein-tyrosine kinase (PTK) family (Lai & Lemke, (1991) Neuron. 6(5):691-704). Designated Tyro3, Tyro 7, and Tyro 12 at that time, the kinase domains of these proteins clearly segregated into a separate family based on sequence conservation (Lai & Lemke, (1991) Neuron. 6(5):691-704). Subsequent isolation of full-length cDNAs by multiple groups confirmed this segregation, and also resulted in multiple names for the receptors. Tyro3, Axl, and Mer are now the consensus, assigned gene designations. An analysis of the mouse and human 'kinomes' indicates that Tyro3, Axl, and Mer constitute the full TAM family. (There are 58 receptor PTK genes in the human and mouse genomes.)

Specific examples of Axl receptor amino acid sequences include, but are not limited to Genbank Accession Nos. NP_001690 (invariant ATP binding Lysine (K) 558) and NP_068713 (as of July 25, 2012). Specific examples of Tyro3 receptor amino acid sequences include, but are not limited to Genbank Accession Nos. NP_006284 (invariant ATP binding Lysine (K) 550), EAW92506, and EAW92507 (as of July 25 2012). Specific examples of Mer receptor amino acid sequences include, but are not limited to Genbank Accession Nos. AAK54121, AAI14918 (invariant ATP binding Lysine (K) 443), and AAI14918 (as of July 25, 2012). The invariant ATP binding site Lysine (K) is located in the sequence VAVKTM.

"TAM receptor activity" includes any biological activity of a TAM receptor, for instance an activity that is enhanced or induced by the binding of a TAM receptor ligand to a TAM receptor. TAM receptor ligands include Protein S and Gas6. Specific examples of Gas6 nucleic acid and amino acid sequence include, but are not limited to Genbank^{™} Nos: NM_000820.1 and NP_000811.1 (as of July 25, 2012). Specific examples of Protein S nucleic acid and amino acid sequences include, but are not limited to Genbank^{™} Nos: Genbank^{™} Nos: NM;_000313.1 and NP_000304.1 (as of July 25, 2012). Exemplary TAM receptor activities include, but are not limited to inhibiting or decreasing IFN-α or IFN-β production in response to infection, inducing TAM autophosphorylation, inhibiting TLR-induced cytokine production, inhibiting TLR-induced stimulation of MAP kinase activation, inhibiting TLR-induced NF-kB activation, and increasing SOCS1 and/or SOCS3 expression.

An "inhibitor of TAM receptor activity" includes any composition that decreases a TAM receptor activity, for example in a cell that expresses a TAM receptor. Examples of a decrease in TAM receptor activity include, but are not limited to an increase in IFN-α or IFN-β secretion (*e.g*., by a cell infected with a virus), a decrease in TAM autophosphorylation, an increase in TLR-induced cytokine production, an increase in TLR-induced stimulation of MAP kinase activation, an increase in TLR-induced NF-kB activation, and a decrease in SOCS1 and/or SOCS3 expression. Exemplary methods for measuring such activity are provided herein.

TAM receptor inhibitors include those molecules that reduce TAM receptor activity, such as those that specifically bind to a Tyro3, Axl, or Mer ligand or extracellular domain and prevent the interaction between the ligand and the receptor or PtdSer in a membrane, molecules that decrease TAM receptor ligand concentration (such as decreasing Protein S and or Gas6 levels using Protein S or Gas6 specific siRNA or antibodies), and molecules that bind to the intracellular domain (*e.g*., a kinase domain or ATP binding site) and prevent signaling of the receptor (and thus significantly reduce or inhibit receptor activation). Exemplary inhibitors include the small molecule inhibitors of TAM (*e.g.,* Axl) kinase activity, as well as antibodies that (a) bind to TAM receptors and block receptor activation, (b) block the interaction of TAM receptors with Gas6 or ProS ligands, (b) block the interaction of Gas6 or ProS ligands with PtdSer in a membrane, or (d) bind to the ligands and prevent them from activating their cognate TAM receptors, and RNAi molecules that significantly decrease or inhibit expression of Tyro3, Axl or Mer.

An "enhancer of TAM receptor activity" includes any composition that increases TAM receptor activity. Examples of an increase in TAM receptor activity include, but are not limited to a an increase in TAM autophosphorylation, a decrease in TLR-induced cytokine production, a decrease in TLR-induced stimulation of MAP kinase activation, a decrease in TLR-induced NF-kB activation, and an increase in SOCS1 and/or SOCS 3 expression. An exemplary enhancer of TAM receptor activity is a PtdSer-containing membrane having a Gas6 and/or Protein S protein bound to the membrane. Exemplary methods for measuring such activity are provided herein.

**Therapeutically effective amount:** An amount of a therapeutic agent (such as a TAM receptor agonist or antagonist), alone or in combination with other agents (such as an anti-viral agent) sufficient to prevent advancement of a disease, to cause regression of the disease, or which is capable of relieving symptoms caused by the disease, such as fever, respiratory symptoms, pain or swelling. In some examples, it is an amount that results in a decrease of symptoms upon viral infection or results in a delay, amelioration, or prevention of a disease associated with infection by a virus. In some examples, it is an amount that results in a decrease of symptoms associated with an autoimmune disease or results in a delay, amelioration, or prevention of a disease associated with autoimmune disease.

The particular dose for a therapeutically effective amount of a particular TAM receptor inhibitor or activator will depend on the particular agent used, the weight, age and condition of the subject to be treated, the drug combination used, and the like. However, such amounts can be determined using methods well known in the art. In a particular example, a therapeutically effective amount of a TAM receptor inhibitor or activator is 0.001 mg/kg to 100 mg/kg for a 70 kg mammal, such as 0.01 to 50 mg/kg, or 1 to 25 mg/kg. In another particular example, a therapeutically effective amount of a TAM receptor inhibitor or activator is 0.001 µg/kg to 100 µg/kg for a 70 kg mammal, such as 0.01 to 50 µg/kg, or 1 to 25 µg/kg.

**Treating a disease:** "Treatment" refers to a therapeutic intervention that ameliorates a sign or symptom of a disease or pathological condition after it has begun to develop, such a sign or symptom of a viral infection or autoimmune disease. Treatment can also induce remission or cure of a condition, such those associated with viral infections or autoimmune disease.

**Preventing a disease** refers to a therapeutic intervention to a subject who does not exhibit signs of a disease or exhibits only early signs for the purpose of decreasing the risk of developing pathology, such that the therapy inhibits or delays the full development of a disease, such as preventing development of a viral infection or autoimmune disease. For example, persons at risk for being exposed to a virus include, but are not limited to, military personnel, medical personnel, travelers, and caregivers of adults and children, as well as those with weakened immune systems, for example, the elderly, people on immunosuppressive drugs, subjects with cancer, and subjects infected with HIV. Thus, for example, TAM receptor antagonists (such as an agent that interferes with the interaction between a TAM ligand and PtdSer) can be administered to such subject to prevent a viral infection.

Treatment and prevention of a disease does not require a total absence of disease. For example, a decrease of at least 20% or at least 50% can be sufficient. The beneficial effect can be evidenced, for example, by a delayed onset of clinical symptoms of the disease in a susceptible subject, a reduction in severity of some or all clinical symptoms of the disease, a slower progression of the disease, a reduction in the viral load, an improvement in the overall health or well-being of the subject, or by other parameters well known in the art that are specific to the particular disease.

### Overview

Viruses must evade or inhibit innate immune responses in order to infect and propagate within their hosts. We have identified a novel mechanism of immune evasion involving viral activation of the TAM family of receptor tyrosine kinases - Tyro3, Axl, and Mer. Many different enveloped viruses display the phospholipid phosphatidylserine on their surface membranes, which allows them to bind to and present the TAM ligands, Gas6 and Protein S. We find that engagement of TAM-ligand-coated virus particles with TAM receptors expressed on the surface of target cells immediately activates the receptors. Surprisingly, membrane-bound TAM ligands activate TAM receptors to a significantly higher level than free, non membrane-bound ligands. This increased agonist activity of TAM ligands bound to virus membranes is present in "bald" virons lacking any viral proteins, and can be significantly attenuated through treatment with Annexin V, a phosphatidylserine-binding protein, demonstrating that binding of Gas6 or Protein S to phosphatidylserine-containing membranes through the gla-domain on the TAM receptor ligands, rather than any viral factors that might be present on the extracellular surface of the membrane, is critical for the increased agonist activity. These results demonstrate that the interaction between lipids and the gla-domain changes ligand properties towards TAM receptors, providing a novel approach to modulate ligand-receptor interaction and function. Activated TAM receptors in turn induce the cytokine signaling inhibitors SOCS1 and SOCS3. These cytoplasmic inhibitors markedly dampen signaling by type I interferons (IFNs), which are among the most potent of anti-viral cytokines. When challenged with virus, dendritic cells deficient in TAM receptors display dramatically elevated type I IFN responses and correspondingly reduced infectivity relative to TAM-expressing cells. The results provided herein illuminate a powerful and very general mechanism of viral immune evasion, and indicate that TAM receptor inhibition is an attractive approach to new broad-spectrum anti-viral therapeutics.

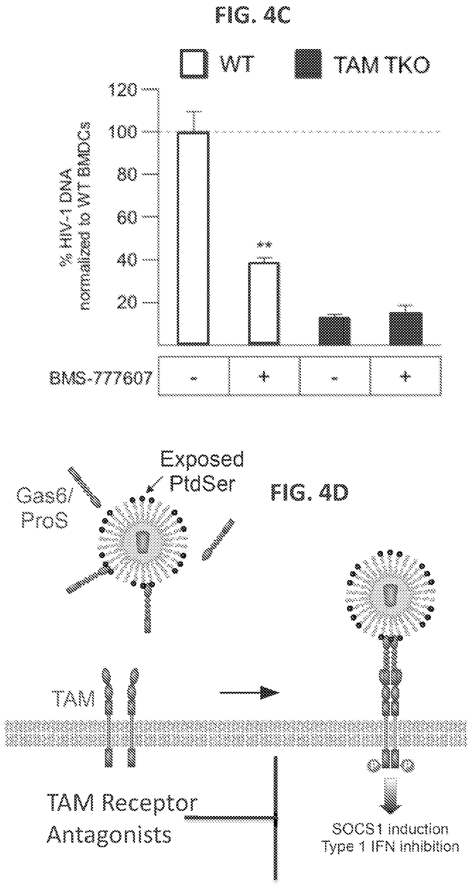

The data herein are consistent with a new model of the role of TAM receptor-ligand interactions during enveloped virus entry (FIG. 4D). Specifically, TAM ligands become immobilized and concentrated on the surface of enveloped virions and act as "super-ligands" to efficiently activate cell surface TAM receptors during virus entry. TAM receptor activation leads to the up-regulation of SOCS gene expression and to the consequent down-regulation of host TLR- and type I IFN signaling pathways. This model provides a general mechanism by which enveloped viruses can interfere with host antiviral defenses and thereby render a target cell more permissive for virus replication. Since the virus infection defect observed with TAM-deficient BMDCs was largely overcome in the presence of interferon α and β neutralizing antibodies, it is proposed that the major role played by these receptors is through inhibiting the cellular innate immune response. Furthermore, data obtained with the BMS-777607 compound indicate a broad spectrum therapeutic approach based upon the use of small molecule antagonists that block virus-mediated TAM receptor activation and the downstream inhibition of type I IFN signaling.

### Methods of Increasing Potency of TAM Activating Agents

The present disclosure provides methods of increasing potency of TAM activating agents, specifically the native TAM ligands Protein S and Gas6, by providing a PtdSer-containing lipid membrane bilayer to complex with the gla-domain of said ligands, thereby increasing their potency with regard to the activation of TAM signaling. In some examples, such methods are used to treat an autoimmune disease in a subject. For example, such methods can include administering to the subject a therapeutically effective amount of a TAM receptor agonist, which includes a PtdSer-containing lipid bilayer membrane (such a liposome or a nanoparticle) with Gas6 and/or Protein S bound to the membrane. Such a method activates signaling from one or more TAM receptors (such as Axl, Mer and/or Tyro3), thereby treating the autoimmune disease. For example, the Gas6 and/or Protein S can bind to the lipid membrane through the PtsdSer-Gla domain interaction. In some examples, the methods further include treating the subject with an immunosuppressant (for example by administering the immunosuppressant to the subject, such as a steroid, glucocorticoid, alkylating agent, methotrexate, and the like). In some examples, the methods further include treating the subject with another drug used to treat autoimmune diseases, such as Interferon (Avonex, Rebif, Betaserone), glatiramer acetate, mitoxantrone, natalizumab (Tysabri), belimumab, LY2127399, Atacicept, rituximab or the like. In some examples, TAM receptor activity is increased by at least 20%, at least 50%, at lest 80%, at least 90%, or even at least 95% (or in some examples by at least 2-fold, at least 3-fold, o rat lest 4-fold), relative to the amount of TAM receptor activity in the absence of the PtdSer-containing lipid bilayer membrane with Gas6 and/or Protein S bound to the membrane.

Exemplary autoimmune diseases that can be treated with such a method, include but are not limited to: amyotriphic lateral sclerosis, celiac disese, rheumatoid arthritis, Hashimoto's thyroiditis, pernicious anemia, inflammatory bowel disease (Crohn's disease and ulcerative colitis), Cushing's syndrome, Guillain-Barre syndrome, transverse myelitis, psoriasis, renal, pulmonary, and hepatic fibroses, Addison's disease, type I diabetes, systemic lupus erythematosus, dermatomyositis, Sjogren's syndrome, multiple sclerosis, myasthenia gravis, Reiter's syndrome, and Grave's disease.

In some examples, the subject is a human, such as one with an autoimmune disease. In some examples, the methods can further include selecting a subject having an autoimmune disease for treatment.

### PtdSer-Containing Membranes with Gas6 and/or Protein S protein

The present disclosure also provides compositions which include a PtdSer-containing membrane having a Gas6 and/or Protein S protein bound to the membrane. In some examples, the PtdSer containing membrane includes a liposome or a nanoparticle. Such compositions are isolated, to distinguish from similar compositions which might be found naturally.

Liposome formulations of drugs are well known in the art and several currently marketed drugs contain liposomes for targeted delivery of drugs. Several manufacturing approaches have been used in the field that are appropriate for the manufacturing of PtdSer-containing liposomes (for example see Mozafari, Cell Mol Biol Lett, 2005; 10(4):711-719 and references therein). Nanoparticle liposomes are liposomes with a diameter range of the liposome particles in the range of 10 to 500 nm, typically between 100 and 500 nm. Methods to prepare nanoparticle liposomes are well known in the art (see, for example, Constantinides et al., Adv Drug Deliv Rev 2008; 60(6):757-767 and references therein).

### Methods of Decreasing Interaction Between TAM Ligands and PtdSer

The present disclosure provides methods of decreasing the interaction between TAM ligands (such as Gas 6 and Protein S) and phosphatidylserine (PtdSer) present on a cell membrane. Such a decrease results in a decrease in TAM receptor activity. In some examples, such methods are used to treat a pathological condition in a subject, such as an infection by an enveloped virus, that is characterized by overactivation of TAM signaling and/or reduction in Type I IFN response. Disrupting the interaction between PtdSer-containing membranes and Gas 6 and/or Protein S leaves the fundamental interaction between Gas6/Protein S and TAM receptors and thus signaling through the TAM receptor system intact, but reduces the potency of the TAM ligands against the receptors. In some examples, such methods are used to treat a pathological condition in a subject where partial reduction, but not full antagonism, of TAM receptor signaling is desirable. For example, such methods can include administering to the subject a therapeutically effective amount of an agent that can disrupt the interaction between Gas6 and/or Protein S with PtdSer-containing lipid bilayer membranes, thereby decreasing TAM signaling. In some examples, the method also includes selecting a subject infected with a virus having PtdSer on the surface of its envelope membrane; or selecting a subject at risk for infection with a virus having PtdSer on the surface of its envelope membrane. In some examples, the methods include further treating the subject with another antiviral agent. In some examples, the method decreases viral infection by at least 2-fold, at least 5-fold, at least 10-fold, at lest 15-fold, or at lest 20-fold, as compared to an absence of therapy.

In some examples, such treatment decreases TAM receptor activity or activation by at least 2-fold, at least 4-fold, at least 5-fold, or at least 10-fold, for example in an immune cell of the infected subject, such as a macrophage, fibroblast, or DC. For example, such methods can in some examples increase type I IFN responses (such as increase in IFN-α or IFN-β production in an immune cell of the infected subject, such as a macrophage, fibroblast, or DC) by at least 5-fold, at least 10-fold, or at least 15-fold (for example as compared to an absence of the treatment). In some examples the type I IFN response includes increased IFNβ and/or IFNα4 mRNA expression. In some examples, such methods can decrease expression of suppressor of cytokine signaling protein (SOCS) 1 and/or SOCS3 by at least 5-fold, at least 10-fold or at least 15-fold (for example as compared to an absence of the treatment).

In one example, the viral infection to be treated or prevented is one caused by an Ebola virus, Marburg virus, vesicular stomatitis virus (VSV), or murine leukemia virus MLV. Examples of other enveloped viruses that can be treated with the methods provided herein include, but are not limited to, enveloped viruses such as members of the following viral families: *Retroviridae* (*e.g.,* HIV (such as HIV1 and HIV2), MLV, SIV, FIV, Human T-cell leukemia viruses 1 and 2, XMRV, and Coltiviruses (such as CTFV or Banna virus)); *Togaviridae* (for example, alphaviruses (such as Ross River virus, Sindbis virus, Semliki Forest Virus, O'nyong'nyong virus, Chikungunya virus, Eastern equine encephalitis virus, Western equine encephalitis virus, Venezuelan equine encephalitis virus) or rubella viruses); *Flaviridae* (for example, dengue viruses or Hepatitis C virus), encephalitis viruses (such as West Nile virus or Japanese encephalitis virus), yellow fever viruses); *Coronaviridae* (for example, coronaviruses such as SARS virus or Toroviruses); *Rhabdoviridae* (for example, vesicular stomatitis viruses, rabies viruses); *Paramyxoviridae* (for example, parainfluenza viruses, mumps virus, measles virus, respiratory syncytial virus, sendai virus, and metopneumovirus); *Orthomyxoviridae* (for example, influenza viruses); *Bunyaviridae* (for example, Hantaan virus, bunya viruses (such as La Crosse virus), phleboviruses, and Nairo viruses); *Hepadnaviridae* (Hepatitis B viruses); *Herpesviridae* (herpes simplex virus (HSV) 1 and HSV-2, varicella zoster virus, cytomegalovirus (CMV), HHV-8, HHV-6, HHV-7, and pseudorabies virus); *Filoviridae* (filoviruses including Ebola virus and Marburg virus) and *Poxviridae* (variola viruses, vaccinia viruses, pox viruses (such as small pox, monkey pox, and Molluscum contagiosum virus), yatabox virus (such as Tanapox and Yabapox)). Non-enveloped viruses can also be treated with the methods provided herein, such as members of the following families: *Calciviridae* (such as strains that cause gastroenteritis); *Arenaviridae* (hemorrhagic fever viruses such as LCMV, Lassa, Junin, Machupo and Guanarito viruses); *Reoviridae* (for instance, reoviruses, orbiviruses and rotaviruses); *Birnaviridae; Parvoviridae* (parvoviruses, such as Human bocavirus adeno-associated virus); *Papillomaviridae* (such as papillomaviruses); *Papovaviridae* (papilloma viruses, polyoma viruses); *Adenoviridae* (adenoviruses); *Picornaviridae* (enteroviruses, enteric viruses, Poliovirus, coxsackieviruses, hepatoviruses, cardioviruses, aptoviruses, echoviruses, hepatitis A virus, Foot and mouth disease virus, and rhinovirus) and *Iridoviridae* (such as African swine fever virus). Other viruses that can be treated using the methods provided herein include unclassified viruses (for example, the etiological agents of Spongiform encephalopathies, the agent of delta hepatitis (thought to be a defective satellite of hepatitis B virus), the agents of non-A, non-B hepatitis (class 1 = internally transmitted; class 2 = parenterally transmitted (for instance, Hepatitis C); calciviruses (such as Norovirus, Norwalk and related viruses); Hepeviruses (such as Hepatitis E, JC and BK viruses) and astroviruses).

In one example, the agent that can disrupt the interaction between Gas6 and/or Protein S with PtdSer is an antibody (or fragment thereof) specific for the Gla-domain of Gas6 and/or Protein S (*e.g.,* see GenBank Accession Nos. NP_000811.1 and NP_000304.2), or an antibody to PtdSer. The term "antibody" refers to an immunoglobulin molecule (or combinations thereof) that specifically binds to, or is immunologically reactive with, a particular antigen, and includes polyclonal, monoclonal, genetically engineered and otherwise modified forms of antibodies, including but not limited to chimeric antibodies, humanized antibodies, heteroconjugate antibodies (*e.g*., bispecific antibodies, diabodies, triabodies, and tetrabodies), single chain Fv antibodies (scFv), polypeptides that contain at least a portion of an immunoglobulin that is sufficient to confer specific antigen binding to the polypeptide, and antigen binding fragments of antibodies. Antibody fragments include proteolytic antibody fragments [such as F(ab')2 fragments, Fab' fragments, Fab'-SH fragments, Fab fragments, Fv, and rIgG], recombinant antibody fragments (such as sFv fragments, dsFv fragments, bispecific sFv fragments, bispecific dsFv fragments, diabodies, and triabodies), complementarity determining region (CDR) fragments, camelid antibodies (see, for example, U.S. Patent Nos. 6,015,695; 6,005,079; 5,874,541; 5,840,526; 5,800,988; and 5,759,808), and antibodies produced by cartilaginous and bony fishes and isolated binding domains thereof (see, for example, International Patent Application No. WO03014161).

A Fab fragment is a monovalent fragment consisting of the VL, VH, CL and CH1 domains; a F(ab')₂ fragment is a bivalent fragment comprising two Fab fragments linked by a disulfide bridge at the hinge region; an Fd fragment consists of the VH and CHI domains; an Fv fragment consists of the VL and VH domains of a single arm of an antibody; and a dAb fragment consists of a VH domain (see, *e.g.,* Ward et al., Nature 341:544-546, 1989). A single-chain antibody (scFv) is an antibody in which a VL and VH region are paired to form a monovalent molecule via a synthetic linker that enables them to be made as a single protein chain (see, *e.g.,* Bird et al., Science, 242: 423-426, 1988; Huston et al., Proc. Natl. Acad. Sci. USA, 85:5879-5883, 1988). Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (see, *e.g.,* Holliger et al., Proc. Natl. Acad. Sci. USA, 90:6444-6448, 1993; Poljak et al., Structure, 2:1121-1123, 1994). A chimeric antibody is an antibody that contains one or more regions from one antibody and one or more regions from one or more other antibodies. An antibody may have one or more binding sites. If there is more than one binding site, the binding sites may be identical to one another or may be different. For instance, a naturally occurring immunoglobulin has two identical binding sites, a single-chain antibody or Fab fragment has one binding site, while a "bispecific" or "bifunctional" antibody has two different binding sites.

As used herein, the term "selectively binds to" refers to the specific binding of one protein to another (for instance, an antibody, fragment thereof, or binding partner to an antigen), wherein the level of binding, as measured by any standard assay (for example, an immunoassay), is statistically significantly higher than the background control for the assay. For example, when performing an immunoassay, controls typically include a reaction well/tube that contain antibody or antigen binding fragment alone (for instance, in the absence of antigen), wherein an amount of reactivity (for instance, non-specific binding to the well) by the antibody or antigen binding fragment thereof in the absence of the antigen is considered to be background.

In some examples, an antibody specifically binds to its target (*e.g*., Gla-domain of Gas6, Gla-domain of Protein S, or PtdSer) with a binding constant that is at least 10³ M⁻¹ greater, 10⁴ M⁻¹ greater or 10⁵ M⁻¹ greater than a binding constant for other molecules in a sample. In some examples, such antibodies (*e.g.*, monoclonal antibody) or fragments thereof has an equilibrium constant (K_{d}) of 1 nM or less. For example, antibodies that bind to a Gla-domain of Gas6, Gla-domain of Protein S, or PtdSer with a binding affinity of at least about 0.1 x 10⁻⁸ M, at least about 0.3 x 10⁻⁸ M, at least about 0.5 x 10⁻⁸ M, at least about 0.75 x 10⁻⁸ M, at least about 1.0 x 10⁻⁸ M, at least about 1.3 x 10⁻⁸ M at least about 1.5 x 10⁻⁸M, or at least about 2.0 x 10⁻⁸ M. Kd values can, for example, be determined by competitive ELISA (enzyme-linked immunosorbent assay) or using a surface-plasmon resonance device such as the Biacore T100, which is available from Biacore, Inc., Piscataway, NJ. Binding can be measured using a variety of methods standard in the art, including, but not limited to: Western blot, immunoblot, enzyme-linked immunosorbant assay (ELISA), radioimmunoassay (RIA), immunoprecipitation, surface plasmon resonance, chemiluminescence, fluorescent polarization, phosphorescence, immunohistochemical analysis, matrix-assisted laser desorptionlionization time-of-flight mass spectrometry, microcytometry, microarray, microscopy, fluorescence activated cell sorting (FACS), and flow cytometry.

In one example, the agent that can disrupt the interaction between Gas6 and/or Protein S with PtdSer is a full length Gla-domain derived from human Gas6 or Protein S sequence, or a fragment thereof. The sequence of the full length Gla-domain derived from human Gas6 is shown in amino acids 39 to 92 of GenBank Accession No. NP_000811.1 (tq flrprqrraf qvfeeakqgh lerecveelc sreearevfe ndpetdyfyp ry), and from human Protein S in amino acids 23-85 of GenBank Accession No. NP_000304.2 (eanflskq qasqvlvrkr ranslleetk qgnlerecie elcnkeeare vfendpetdy fypky). In some examples, the fragment of the full length Gla-domain consists of at least 8, at least 10, at least 15, at least 20, at least 30, at least 30 or at least 40 contiguous amino acids of amino acids 39 to 92 of GenBank Accession No. NP_000811.1 or amino acids 23-85 of GenBank Accession No. NP_000304.2.

In one example, the agent that can disrupt the interaction between Gas6 and/or Protein S with PtdSer is a natural or artificial peptide that binds to PtdSer, for example Annexin V or a fragment of Annexin V, with high affinity.

### Pharmaceutical Compositions and Administration

The agents use to modulate the interaction between TAM receptor ligands and PtdSer in membranes used in the methods described herein can be formulated in a variety of ways depending on the location and type of disease to be treated. For example, such agents can disrupt the PtdSer-gla interactions (for example to inhibit TAM signaling), or activate TAM signaling by increasing the activity of PtdSer-Protein S/Gas 6 complexes. Pharmaceutical compositions are thus provided for both local (for instance, topical or inhalational) use and for systemic use. Therefore, the disclosure includes within its scope pharmaceutical compositions including at least one agent that modulates the interaction between TAM receptor ligands and PtdSer in membranes (*e.g*., one, two or three such agents) formulated for use in human or veterinary medicine. While the agents that modulate the interaction between TAM receptor ligands and PtdSer in membranes typically will be used to treat human subjects, they also can be used to treat similar or identical diseases in other vertebrates, such other primates, dogs, cats, horses, and cows.

Pharmaceutical compositions that include at least one agent that modulates the interaction between TAM receptor ligands and PtdSer in membranes as described herein as an active ingredient, or that include both an agent that modulates the interaction between TAM receptor ligands and PtdSer in membranes and an additional therapeutic agent (such as an anti-viral or immunosuppressant) as active ingredients, can be formulated with an appropriate solid or liquid carrier, depending upon the particular mode of administration chosen. A suitable administration format can best be determined by a medical practitioner for each subject individually. Various pharmaceutically acceptable carriers and their formulation are described in standard formulation treatises, for instance, Remington's Pharmaceutical Sciences by E. W. Martin Mack Publishing Co., Easton, PA, 15th Edition (1975). See also Wang & Hanson (1988) Journal of Parenteral Science and Technology, Technical Report No. 10, Supp. 42: 2S.

The dosage form of the pharmaceutical composition is determined by the mode of administration chosen. For instance, in addition to injectable fluids, inhalational, transdermal, rectal, vaginal, and oral formulations can be employed. Inhalational preparations can include aerosols, particulates, and the like. In general, the goal for particle size for inhalation is about 1µm or less in order that the pharmaceutical reach the alveolar region of the lung for absorption. Oral formulations can be liquid (for instance, syrups, solutions, or suspensions), or solid (for instance, powders, pills, tablets, or capsules). For solid compositions, conventional non-toxic solid carriers can include pharmaceutical grades of mannitol, lactose, starch, or magnesium stearate. Actual methods of preparing such dosage forms are known, or will be apparent, to those of ordinary skill in the art.

In one embodiment, a pharmacological composition is provided that includes at least one agent that modulates the interaction between TAM receptor ligands and PtdSer in membranes (such as an agent that increases or decreases TMA signaling) and a pharmacologically acceptable carrier. Pharmacologically acceptable carriers (for instance, physiologically or pharmaceutically acceptable carriers) are well known in the art. A suitable pharmacological composition can be formulated to facilitate the use of agents that modulate the interaction between TAM receptor ligands and PtdSer in membranes *in vivo.* Such a composition can be suitable for delivery of the active ingredient to any suitable host, such as a patient for medical application, and can be manufactured in a manner that is itself known, for instance, by means of conventional mixing dissolving, granulating, dragee-making, levigating, emulsifying, encapsulating, entrapping, or lyophilizing processes.

The compositions or pharmaceutical compositions can be administered by any route, including parenteral administration, for example, intravenous, intraperitoneal, intramuscular, intraperitoneal, intrathecal, or intra-articular injection or infusion, or by sublingual, oral, topical, rectal, vaginal, intra-nasal, or transmucosal administration, or by pulmonary inhalation. When agents that modulate the interaction between TAM receptor ligands and PtdSer in membranes are provided as parenteral compositions, for instance, for injection or infusion, they are generally suspended in an aqueous carrier, for example, in an isotonic buffer solution at a pH of about 3.0 to about 8.0, for example at a pH of about 3.5 to about 7.4, 3.5 to 6.0, or 3.5 to about 5.0. Useful buffers include sodium citrate-citric acid and sodium phosphate-phosphoric acid, and sodium acetate/acetic acid buffers.

For oral administration, the pharmaceutical compositions that include one or more agents that modulate the interaction between TAM receptor ligands and PtdSer in membranes can take the form of, for example, tablets or capsules prepared by conventional means with pharmaceutically acceptable excipients such as binding agents (for instance, pregelatinised maize starch, polyvinyl pyrrolidone or hydroxypropyl methylcellulose); fillers (for instance, lactose, microcrystalline cellulose or calcium hydrogen phosphate); lubricants (for instance, magnesium stearate, talc or silica); disintegrants (for instance, potato starch or sodium starch glycolate); or wetting agents (for instance, sodium lauryl sulphate). The tablets can be coated by methods well known in the art. Liquid preparations for oral administration can take the form of, for example, solutions, syrups or suspensions, or they can be presented as a dry product for constitution with water or other suitable vehicle before use. Such liquid preparations can be prepared by conventional means with pharmaceutically acceptable additives such as suspending agents (for instance, sorbitol syrup, cellulose derivatives or hydrogenated edible fats); emulsifying agents (for instance, lecithin or acacia); non-aqueous vehicles (for instance, almond oil, oily esters, ethyl alcohol or fractionated vegetable oils); and preservatives (for instance, methyl or propyl-p-hydroxybenzoates or sorbic acid). The preparations can also contain buffer salts, flavoring, coloring, and sweetening agents as appropriate.

For administration by inhalation, the agents that modulate the interaction between TAM receptor ligands and PtdSer in membranes for use according to the present disclosure are conveniently delivered in the form of an aerosol spray presentation from pressurized packs or a nebulizer, with the use of a suitable propellant, for instance, dichlorodifluoromethane, trichlorofluoromethane, dichlorotetrafluoroethane, carbon dioxide or other suitable gas. In the case of a pressurized aerosol, the dosage unit can be determined by providing a valve to deliver a metered amount. Capsules and cartridges for use in an inhaler or insufflator can be formulated containing a powder mix of the compound and a suitable powder base such as lactose or starch.

Pharmaceutical compositions that include at least agent that modulates the interaction between TAM receptor ligands and PtdSer in membranes as described herein as an active ingredient normally will be formulated with an appropriate solid or liquid carrier, depending upon the particular mode of administration chosen. The pharmaceutically acceptable carriers and excipients useful in this disclosure are conventional. For instance, parenteral formulations usually include injectable fluids that are pharmaceutically and physiologically acceptable fluid vehicles such as water, physiological saline, other balanced salt solutions, aqueous dextrose, glycerol or the like. For solid compositions (for instance, powder, pill, tablet, or capsule forms), conventional non-toxic solid carriers can include, for example, pharmaceutical grades of mannitol, lactose, starch, or magnesium stearate. If desired, the pharmaceutical composition to be administered can also contain minor amounts of non-toxic auxiliary substances, such as wetting or emulsifying agents, preservatives, and pH buffering agents and the like, for example sodium acetate or sorbitan monolaurate. Actual methods of preparing such dosage forms are known, or will be apparent, to those skilled in the art.

In order to increase the desired response in a subject, a therapeutically effective amount of one or more agents that modulate the interaction between TAM receptor ligands and PtdSer in membranes (alone or in combination with other agents) is administered to the subject. An effective amount of an agent that modulates the interaction between TAM receptor ligands and PtdSer in membranes can be administered in a single dose, or in multiple doses. For example, in some embodiments, such agents are administered periodically after the initial administration, for example, twice a day or more. In other embodiments, the agent is administered as a continuous infusion. Agents that modulate the interaction between TAM receptor ligands and PtdSer in membranes can be injected once, for example, or they can be injected in divided doses two or more times, for example monthly, weekly, daily, or 2-4 or more times daily.

In some examples, agents that modulate the interaction between TAM receptor ligands and PtdSer in membranes are administered for short periods of time, to decrease undesired side effects that may result from such long-term administration. Therefore, in particular examples, agents that modulate the interaction between TAM receptor ligands and PtdSer in membranes are administered for a period of no more than 30 days, no more than 14 days, no more than 7 days, or no more than 3 days, such as a period of 1-30 days, 1-14 days, 1-5 days, 7-14 days, or 3-7 days. In other examples, agents that modulate the interaction between TAM receptor ligands and PtdSer in membranes are administered for longer periods of time, but under conditions that decrease undesired side effects that may result from such long-term administration. Therefore, in particular examples, agents that modulate the interaction between TAM receptor ligands and PtdSer in membranes are administered at a dose below the IC₅₀ of the agent, such as a dose that is at least 10%, at least 25%, at least 40%, at least 60% or at least 80% less than the IC₅₀ for the agent, for example, for a period of at least 30 days, at least 60 days, at least 120 days, or at least 365 days, such as a period of 30 to 120 days, 30 to 200 days, or indefinitely.

In one embodiment, the agents that modulate the interaction between TAM receptor ligands and PtdSer in membranes are administered locally, such as by topical application or intradermal administration. In other embodiments, the administration of is systemic. In one embodiment, the agent is administered systemically, such as by intravenous injection, intramuscular injection, or subcutaneous injection. Oral, intravenous, intra-arterial, subcutaneous, intra-peritoneal, intramuscular, inhalational, and even rectal or vaginal administration is contemplated.

The dosage for agents that modulate the interaction between TAM receptor ligands and PtdSer in membranes may vary depending on the particular agent, mode of administration, condition of the subject, age of the subject, or weight of the subject. However, appropriate dosages can be determined by a skilled clinician. In particular examples, an agent that modulates the interaction between TAM receptor ligands and PtdSer in membranes is administered at 0.001 mg/kg to 100 mg/kg for a 70 kg mammal, such as 0.01 to 50 mg/kg, or 1 to 25 mg/kg. In another particular example, a therapeutically effective amount of such an agent is 0.001 µg/kg to 100 µg/kg for a 70 kg mammal, such as 0.01 to 50 µg/kg, or 1 to 25 µg/kg. In a specific example, an agent that modulates the interaction between TAM receptor ligands and PtdSer in membranes is administered at a dose of about 50 to 1000 mg/day for adult patients, such as about 100 to 800 mg/day, 200 to 600 mg/day, for example 400 or 600 mg/day for adult patients.

In particular embodiments, agents that modulate the interaction between TAM receptor ligands and PtdSer in membranes is administered in conjunction with one or more other therapeutic agents (such as antivirals or immunosuppressants) in therapeutically effective amounts. Administration of the agents that modulate the interaction between TAM receptor ligands and PtdSer in membranes can occur prior to administration of the other therapeutic agents, substantially contemporaneously with the other agents, or after administration of the other agents.

Anti-viral compounds, include, but are not limited to, zidovudine, didanosine, zalcitabine, stavudine, lamivudine, abacavir, tenofovir, nevirapine, delavirdine, efavirenz, saquinavir, ritonavir, indinavir, nelfinavir, saquinavir, amprenavir, and lopinavir. Anti-infectious agents also include hyper-immune globulin. Modes of administration and dosages can be determined by a skilled artisan and are routine.

In some examples, an agent that decreases the interaction between TAM receptor ligands and PtdSer in membranes is administered with one or more other agents that stimulate the immune system, such as IFNs, cytokines, interleukins, or other agents that increase cytokine production (for example to treat a viral infection)

In some examples, an agent that increases the interaction between TAM receptor ligands and PtdSer in membranes is administered with one or more other agents that suppress the immune system, such as glucocorticoids, methotrexate, alkylating agents, IL-2 antibodies, CD25 antibodies, CD3 antibodies, ciclosporin, interferon, TNF binding proteins, or other agents that suppress the immune system or are used to treat autoimmune diseases, for example Interferon (Avonex, Rebif, Betaserone), glatiramer acetate, mitoxantrone, natalizumab (Tysabri), belimumab, LY2127399, Atacicept, rituximab or the like.

### Classifying a Virus as Susceptible to anti-TAM therapy

The present disclosure also provides methods for classifying a virus as susceptible to anti-TAM therapy (for example to determine if a particular virus will be susceptible to anti-TAM therapy (such as a small molecule inhibitor of TAM intracellular signaling), such as a virus in a subject). In particular examples the method includes determining whether the virus comprises PtdSer on the envelope surface and/or determining whether the virus induces TAM signaling when in contact with a TAM ligand (such as Gas 6 or Protein S). For example, Annexin V is known to bind to apoptotic cells through binding of exposed PtdSer lipids on the surface of cells. Annexin V binding can also be used to quantify the level of PtdSer on a virus membrane. In another example, a monoclonal antibody specific for PtdSer is used, such as mAb 1H6 (see Mourdjeva et al., Apoptosis, 10:209-17, 2005). The value or amount (such as a qualitative or quantitative amount) of PtdSer on the envelope surface and/or the level of TAM signaling when in contact with a TAM ligand is compared with a value for a virus with a known amount of PtdSer on the envelope surface and/or a virus known to induce TAM signaling. This thereby classifies the virus as susceptible or resistant to anti-TAM therapy. For example, a virus with PtdSer on the envelope surface and/or one that can induce TAM signaling is one that is classified to be susceptible to anti-TAM therapy. In contrast, a virus with no detectable PtdSer on the envelope surface and/or one that cannot induce TAM signaling is one that is classified to be resistant to anti-TAM therapy.

In one example, the method determines whether a virus is susceptible to anti-TAM therapy, such as a small molecule inhibitor of TAM intracellular signaling. In some embodiments, TAM receptor inhibitors are small molecule inhibitors that bind to an ATP binding site of Tyro3, Axl, or Mer. In some examples, small molecule inhibitors that bind to an intracellular kinase domain (such as an ATP binding site) of Tyro3, Axl, or Mer, can be used to decrease the biological activity of a TAM receptor in a cell. In particular examples, the small molecule inhibitor is membrane permeable. Several small molecule TAM receptor inhibitors are known, for instance AXL-1, AXL-2, AXL-3, AXL-4, AXL-5, AXL-6, AXL-7, AXL-8, AXL-9, MP470, BMS-777,607 and SGI-AXL-277. Other small molecule TAM receptor inhibitors can be obtained, for example, from Rigel Pharmaceuticals, Inc., San Francisco, CA and SuperGen, Inc., Dublin, CA. Other specific examples of TAM receptor inhibitors can be found in PCT Publication Nos: WO07030680A3, WO06052936A3, WO04092735A3, WO07056151A WO08083354, WO12028332, WO09127417, WO 11146313, WO13052417, U.S. Patent Publication Nos: US20070142402, US2012088768, US2012015937, US2011281846, US2011183986, US2011105512, US201110511, US2011098274, US2011092502, US2011082131, US2011071133, and European Patent Applications Nos. EP2476679, EP2609091 (all hereby incorporated by reference). In some examples, the AXL inhibitor is a triazole derivative. Examples of AXL inhibitors are disclosed in U.S. Patent Publication 2007/0213375, filed September 13, 2007, US Patent Publication 20080153815, filed October 10 2007, which are incorporated herein by reference in their entirety. In certain examples, the AXL inhibitor is a triazole derivative with one of the following general structures: 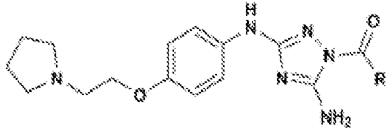 or 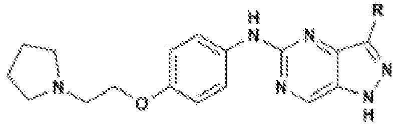 or 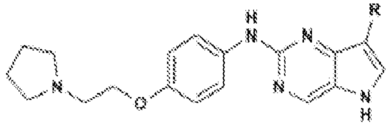 wherein R can be H or CH₃.

In addition to the known TAM receptor inhibitors, higher potency inhibitors can be generated by chemical modification of the existing inhibitors. For instance, the known compounds generally work in the low micromolar range, however chemical modification makes them, in some embodiments, more potent and more specific. In one embodiment, QSAR analysis is performed using the solved Kinase Domain Crystal Structure of MERTK. Axl and Tyro3 kinases also may be modeled upon this crystal structure (see, for instance, Walker, Huang, Finerty Jr., Weigelt, Sundstrom, Arrowsmith, Edwards, Bochkarev, Dhe-Paganon, Human Proto-oncogene Tyrosine-protein Kinase MER (available on the world wide web at www.rcsb.org/pdb/explore.do?structureId=3BRB-; PDB (protein data base) 2P0C). These more potent compositions will have lower IC₅₀ values.

In one example, the small molecule inhibitor of TAM intracellular signaling is R428, also known as BGB324 (see Holland et al., Cancer Research 2010; 70(4):1544-1554 and WO2010/083465 to Hitoshi et al.). The structure of R428/BGB324 is shown below: 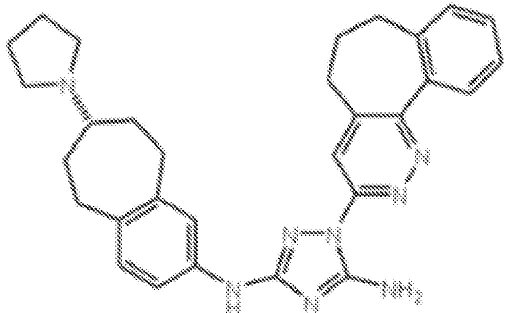 Structural variants of R428 are provided in WO2010/083465, and such variants are encompassed by this disclosure to the extent they inhibit TAM intracellular signaling.

In one example, the small molecule inhibitor of TAM intracellular signaling is BMS-777607, shown in the structure below.

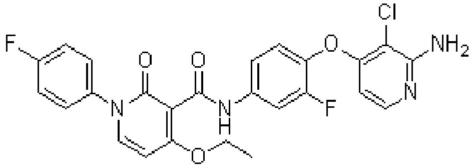

### Methods of Identifying Agents that Blocks Virus Infectivity

The disclosure provides methods for identifying an agent that blocks virus infectivity. Such methods can include contacting one or more test agents with a virus and with an immune cell (such as a dendritic cell or macrophage), determining or measuring TAM receptor activation (such as activation of Tyro3, Axl, and/or Mer) in the presence and absence of the test agent(s), and determining that the test agent(s) blocks virus activity when decreased TAM receptor activation is observed or determining that the test agent(s) does not block virus activity when decreased TAM receptor activation is not observed. For example, if the test agent increases type I IFN (*e.g.,* IFN-α or IFN-β) production by the cell or increases production of an IRF (*e.g.,* IRF3, IRF5, or IRF7), this indicates that the test agent(s) blocks virus activity. For example, detection of increased type I IFN (*e.g.,* IFN-α or IFN-β) or IRF production by the cell in the presence of the test agent (such as an increase of at least 1.5-fold, 2-fold, 5-fold, 10-fold, 20-fold, 40-fold, 50-fold, or 60-fold) relative to a control level representing type I IFN or IRF production by the cells in the absence of the test agent indicates that the test agent blocks virus activity and can be used to treat a viral infection. For example, if the cells were infected with influenza virus and the test agent significantly increased type I IFN or IRF production in such cells, this indicates that the test agent is an anti-influenza agent.

### 1. Cells

Cells that can be used in such an assay include cells that express both a TAM receptor and a cytokine receptor (*e.g.,* type I IFN receptors), such as immune cells that express TAM and type I IFN receptors, for example macrophages and DCs. The TAM receptor and cytokine receptor can be endogenous to the cell or exogenous to the cell (*e.g.,* expressed from a recombinant nucleic acid encoding the protein). In some examples, such cells are primary cells (*e.g.,* directly isolated from a mammalian subject, such as a human or veterinary subject). In other examples, such cells are cell lines, such as those available from American Type Culture Collection, Manassas, VA (*e.g.,* THP-1). In some examples, the cell has substantially no endogenous TAM receptor. Cells expressing exogenous TAM receptor can be, for example, transiently or stably transfected with an expression vector encoding a TAM receptor polypeptide.

The cells are incubated under conditions that permit the pathogen to enter and infect the cell (*e.g.*, allow bacterial or viral replication). Such methods are routine in the art, and will vary depending on the pathogen. For example, cells can be cultured in an appropriate culture medium at 37°C. In some examples, the cell is infected with the virus prior to incubation with the test agent, such as incubation at 37°C for at least 1 hour, at least 8 hours, at least 12 hours, at least 24 hours, at least 48 hours, or at least 72 hours prior to adding the test agent. Such incubation gives the virus time to enter the cells and begin replication prior to adding the test agent. The time points can be selected based on the virus used. Cells can be infected with any target virus, such as those provided herein. For example, if one wanted to identify an anti-vesicular stomatitis virus agent, the cells can be infected with vesicular stomatitis virus.

### 2. Test agents

The conditions also permit the test agent to interact with (*e.g*., specifically bind to) a TAM receptor ligand (*e.g*., Gas6 or ProS), a PtdSer on the membrane of the virus, a TAM receptor binding domain (*e.g*., Tyro3, Axl, or Mer extracellular binding domain), or enter the cell and bind to a Tyro3, Axl, or Mer intracellular kinase domain (*e.g*., ATP binding site). Exemplary test agents that can be used with such methods include any substance or any combination of substances that is useful for achieving an end or result; for example, a substance or combination of substances useful for increasing type I IFN production (*e.g*., IFN-α or IFN-β) and/or IFF production to levels useful for treating an infection. Any agent that has potential (whether or not ultimately realized) to modulate any feature of the TAM receptor pathways disclosed herein is contemplated for use in the methods of this disclosure. For example, contemplated are agents that have potential to, in immune cells, increase type I IFN (*e.g.,* IFN-α or IFN-β) mRNA or protein expression, decrease an interaction between a TAM receptor and one of its ligands, decrease an interaction between an intracellular TAM receptor domain and ATP or other regulatory protein that can activate the TAM receptor, or decrease an activity of a TAM receptor.

Exemplary agents include, but are not limited to, peptides such as, for example, soluble peptides, including but not limited to members of random peptide libraries (see, *e.g.,* Lam et al., Nature, 354:82-84, 1991; Houghten et al., Nature, 354:84-86, 1991), and combinatorial chemistry-derived molecular library made of D- and/or L-configuration amino acids, phosphopeptides (including, but not limited to, members of random or partially degenerate, directed phosphopeptide libraries; see, *e.g.,* Songyang et al., Cell, 72:767-778, 1993), antibodies (including, but not limited to, polyclonal, monoclonal, humanized, anti-idiotypic, chimeric or single chain antibodies, and Fab, F(ab')₂ and Fab expression library fragments, and epitope-binding fragments thereof specific for a TAM receptor or ligand), small organic or inorganic molecules (such as, so-called natural products or members of chemical combinatorial libraries), molecular complexes (such as protein complexes), or nucleic acids (*e.g*., siRNAs specific for a TAM receptor).

In one example, the test agent is one that can disrupt the interaction between Gas6 and/or Protein S with PtdSer, such as an antibody (or fragment thereof) specific for the Gla-domain of Gas6 and/or Protein S, or an antibody to PtdSer, a full length Gla-domain derived from human Gas6 or Protein S sequence, or a fragment thereof, or a natural or artificial peptide binding PtdSer.

In one example, derivatives of MP470, SGI-AXL-277, AXL-1, AXL-2, AXL-3, AXL-4, AXL-5, AXL-6, AXL-7, AXL-8, AXL-9, BMS-777607 or R428 are screened for their ability to increase type I IFN and/or IRF production and thus serve as potential antimicrobial agents. For example, derivatives with one of the following general structures are screened for their ability to increase type I IFN production via inhibiting the intracellular kinase activity of a TAM receptor and thus serve as potential antimicrobial agents: 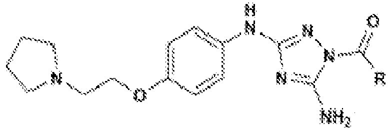 or 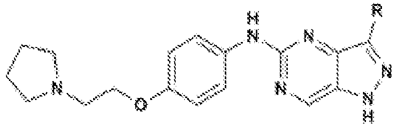 or

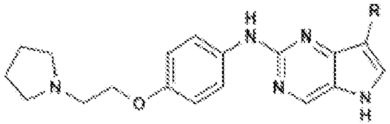

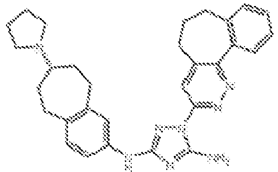

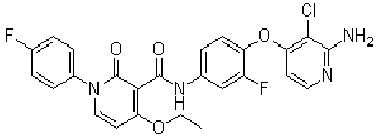

In some examples, the R is H or CH₃.

Libraries (such as combinatorial chemical libraries) useful in the disclosed methods include, but are not limited to, peptide libraries (see, *e.g.,* U.S. Pat. No. 5,010,175; Furka, Int. J. Pept. Prot. Res., 37:487-493, 1991; Houghton et al., Nature, 354:84-88, 1991; PCT Publication No. WO 91/19735), encoded peptides (*e.g.,* PCT Publication WO 93/20242), random bio-oligomers *(e.g.,* PCT Publication No. WO 92/00091), benzodiazepines (*e.g*., U.S. Pat. No. 5,288,514), diversomers such as hydantoins, benzodiazepines and dipeptides (Hobbs et al., Proc. Natl. Acad. Sci. USA, 90:6909-6913, 1993), vinylogous polypeptides (Hagihara et al., J. Am. Chem. Soc., 114:6568, 1992), nonpeptidal peptidomimetics with glucose scaffolding (Hirschmann et al., J. Am. Chem. Soc., 114:9217-9218, 1992), analogous organic syntheses of small compound libraries (Chen et al., J. Am. Chem. Soc., 116:2661, 1994), oligocarbamates (Cho et al., Science, 261:1303, 1003), and/or peptidyl phosphonates (Campbell et al., J. Org. Chem., 59:658, 1994), nucleic acid libraries (see Sambrook et al. Molecular Cloning, A Laboratory Manual, Cold Springs Harbor Press, N.Y., 1989; Ausubel et al., Current Protocols in Molecular Biology, Green Publishing Associates and Wiley Interscience, N.Y., 1989), peptide nucleic acid libraries (see, *e.g.,* U.S. Pat. No. 5,539,083), antibody libraries (see, *e.g.,* Vaughn et al., Nat. Biotechnol., 14:309-314, 1996; PCT App. No. PCT/US96/10287), carbohydrate libraries (see, *e.g.,* Liang et al., Science, 274:1520-1522, 1996; U.S. Pat. No. 5,593,853), small organic molecule libraries (see, *e.g*., benzodiazepines, Baum, C&EN, Jan 18, page 33, 1993; isoprenoids, U.S. Pat. No. 5,569,588; thiazolidionones and methathiazones, U.S. Pat. No. 5,549,974; pyrrolidines, U.S. Pat. Nos. 5,525,735 and 5,519,134; morpholino compounds, U.S. Pat. No. 5,506,337; benzodiazepines, 5,288,514) and the like.

Libraries useful for the disclosed screening methods can be produced in a variety of manners including, but not limited to, spatially arrayed multipin peptide synthesis (Geysen, et al., Proc. Natl. Acad. Sci., 81(13):3998-4002, 1984), "tea bag" peptide synthesis (Houghten, Proc. Natl. Acad. Sci., 82(15):5131-5135, 1985), phage display (Scott and Smith, Science, 249:386-390, 1990), spot or disc synthesis (Dittrich et al., Bioorg. Med. Chem. Lett., 8(17):2351-2356, 1998), or split and mix solid phase synthesis on beads (Furka et al., Int. J. Pept. Protein Res., 37(6):487-493, 1991; Lam et al., Chem. Rev., 97(2):411-448, 1997).

Libraries may include a varying number of compositions (members), such as up to about 100 members, such as up to about 1000 members, such as up to about 5000 members, such as up to about 10,000 members, such as up to about 100,000 members, such as up to about 500,000 members, or even more than 500,000 members.

### 3. Exemplary assays

In some examples, determining whether the test agent increases type I IFN (*e.g.,* IFN-α or IFN-β) production by the cell includes determining a control level of type I IFN (*e.g.,* IFN-α or IFN-β) production by the infected cell before contacting (*e.g*., incubating or treating) the cell with the test agent, contacting the infected (or soon to be infected) cell with the test agent, and determining whether contacting the cell with the test agent increases type I IFN (*e.g.,* IFN-α or IFN-β) production by the cell as compared to the control level of type I IFN (*e.g.,* IFN-α or IFN-β) production. In this example, increased type I IFN (*e.g.,* IFN-α or IFN-β) production by the cell in the presence of the test agent (such as an increase of at least 1.5-fold, 2-fold, 5-fold, 10-fold, 20-fold, 40-fold, 50-fold, or 60-fold) relative to the control level indicates that the test agent is an antiviral agent for the tested virus. In some examples, IRF production is also assayed, wherein increases in IRF production by the cell in the presence of the test agent (such as an increase of at least 1.5-fold, 2-fold, 5-fold, 10-fold, 20-fold, 40-fold, 50-fold, or 60-fold) relative to the control level indicates that the test agent is an antiviral agent.

In other examples, determining whether the test agent increases type I IFN (*e.g.,* IFN-α or IFN-β) production by the cell includes contacting the infected cell with the test agent, measuring and in some examples quantifying type I IFN produced by the cell, comparing the type I IFN produced to a control or reference value (or range of values expected for a particular condition), and determining whether contacting the cell with the test agent increases type I IFN (*e.g.,* IFN-α or IFN-β) production by the cell. For example, if the amount of type I IFN produced by the cell is substantially increased relative to a control or reference value for type I IFN production by the same cell in the absence of the test agent, this indicates that the agent is an antiviral agent for the virus tested. In this example, increased type I IFN (*e.g.,* IFN-α or IFN-β) production by the cell in the presence of the test agent (such as an increase of at least 1.5-fold, 2-fold, 5-fold, 10-fold, 20-fold, 40-fold, 50-fold, or 60-fold) relative to the control level indicates that the test agent is an antiviral agent for the virus tested. Similarly, if the amount of type I IFN produced by the cell is substantially similar or increased relative to a control or reference value for type I IFN production by the same cell in the presence of a known antiviral agent, this indicates that the test agent is an antiviral agent. Alternatively, if the amount of type I IFN produced by the cell is substantially similar relative to a control or reference value for type I IFN production by the same cell in the absence of the test agent or known antiviral, this indicates that the test agent is not an antiviral agent. In some examples, IRF production is also assayed and compared to an IRF control as described for type I IFN.

In one convenient embodiment, high throughput screening methods involve providing a combinatorial chemical or peptide library containing a large number of potential therapeutic compounds (*e.g*., antimicrobials). Such combinatorial libraries are then screened in one or more assays as described herein to identify those library members (particularly chemical species or subclasses) that display a desired characteristic activity (such as, increasing type I IFN (*e.g.,* IFN-α or IFN-β) and/or IRF mRNA or protein expression (such as an increase of at least 20%, at least 50%, at least 80%, or at least 95% relative to the absence of the test agent), decreasing an interaction between a TAM receptor and one of its ligands (such as a decrease of at least 20%, at least 50%, at least 80%, or at least 95% relative to the absence of the test agent), decreasing an interaction between an intracellular TAM receptor domain and ATP or other regulatory protein that can activate the TAM receptor (such as a decrease of at least 20%, at least 50%, at least 80%, or at least 95% relative to the absence of the test agent), or decreasing an activity of a TAM receptor (such as a decrease of at least 20%, at least 50%, at least 80%, or at least 95% relative to the absence of the test agent)). The compounds thus identified can serve as conventional "lead compounds" or can themselves be used as potential or actual therapeutics. In some instances, pools of candidate agents may be identified and further screened to determine which individual or subpools of agents in the collective have a desired activity.

In some cell-based method embodiments described here and throughout the specification, test cells or test agents can be presented in a manner suitable for high-throughput screening; for example, one or a plurality of test cells can be seeded into wells of a microtiter plate, and one or a plurality of test agents can be added to the wells of the microtiter plate. Alternatively, one or a plurality of test agents can be presented in a high-throughput format, such as in wells of microtiter plate (either in solution or adhered to the surface of the plate), and contacted with one or a plurality of test cells under conditions that, at least, sustain the test cells. Test agents can be added to test cells at any concentration that is not toxic to the cells. It is expected that different test agents will have different effective concentrations. Thus, in some methods, it is advantageous to test a range of test agent concentrations.

Expression of a type I IFN-encoding nucleic acid (such as, an IFN-α or IFN-β gene or transcript) or polypeptide (as well as IRF nucleic acids and peptides) can be measured by any method known in the art. For example, the absolute or relative levels of a type I IFN or IRF transcript or polypeptide can be measured by standard techniques, such as, for RNA, Northern blot, PCR (including RT-PCR or q-PCR), *in situ* hybridization, or nucleic acid microarray, or, for protein, Western blot, antibody array, or immunohistochemistry. In some methods, the expression of a type I IFN or IRF mRNA can also be increased by increased stability of the mRNA. In particular methods, the expression of a type I IFN-encoding nucleic acid (such as, an IFN-α or IFN-β gene or transcript) or polypeptide (or IRF nucleic acid or peptide) is increased when its level or activity is at least 10%, at least 20%, at least 30%, at least 50%, at least 100% or at least 250% higher than control measurements of the same indicator (*e.g*., in the same test system prior to addition of a test agent, or in a comparable test system in the absence of a test agent).

In some examples, type I IFN production or IRF is assayed by detecting a change (*e.g.,* an increase) in the expression of a type I IFN- (*e.g.,* IFN-α or IFN-β) or IRF- (*e.g.,* IRF3, IRF5, or IRF) encoding nucleic acid. Expression of a gene or gene product (*e.g*., transcript or protein) can be determined using any expression system capable of expressing a type I IFN (*e.g.,* IFN-α or IFN-β) or IRF polypeptide or transcript (such as, a cell, tissue, or organism, or *in vitro* transcription or translation systems). In some embodiments, cell-based assays are performed. Non-limiting exemplary cell-based assays may involve test cells such as, cells (including cell lines) that normally express a type I IFN- (*e.g.,* IFN-α or IFN-β) or IRF gene, its corresponding transcript(s) and/or type I IFN- (*e.g.,* IFN-α or IFN-β) or IRF protein(s), or cells (including cell lines) that have been transiently transfected or stably transformed with a reporter construct driven by a regulatory sequence of a type I IFN- (*e.g.,* IFN-α or IFN-β) or IRF gene.

Methods of detecting type I IFNs and IRFs are well known in the art. In one example, cells expressing a TAM receptor are cultured in the presence of a pathogen for 1 to 48 hours and subsequently treated with test media containing the test agent(s), for instance, for 1 to 12 hours (*e.g.,* 4 to 8 hours; 0 hours for a negative control) at 37°C. Type I IFN and/or IRF production is then measured. Cytokine assays are well known in the art. For example, cytokine assays are manufactured by Assay Designs, Inc, Ann Arbor, Michigan; AssayGate, Inc., Ijamsville, MD; and Panomics, Inc., Fremont, CA. Exemplary assays include analyzing the supernatant or cells for the presence of a type I IFN (or IRF) using ELISA or analyzing the cell lysate for the presence of type I IFN or IRF nucleic acids using the appropriate primers/probes with qPCR (*e.g*., qRT-PCR). An increase in Type I IFN or IRF production by the cells incubated in test media relative to the control level of Type I IFN or IRF production by cells not incubated in the test media indicates that the test agent inhibits TAM receptor activity. In some examples, an increase of at least 20-fold, at least 25-fold, at least 35-fold, at least 40-fold, at least 45-fold, or even at least 50-fold relative to a control measurement indicates that the test agent is an antiviral.

Inhibiting TAM receptors to increase type I IFN production has advantageous effects as described herein. Thus, it may be beneficial, in some instances, to further determine whether the effect(s) of an agent identified in some method embodiments is (are) antiviral *in vivo.* Thus, it further may be beneficial (although optional) to further screen agents identified in some method embodiments for their potential to treat or prevent a viral infection in a subject; for example, by administering a candidate agent to a subject infected with a pathogen (such as an animal model for the target pathogen, such as a mouse, rat, rabbit, pig, or monkey model) and determining whether the infection is treated by the candidate agent (such as by a decrease in symptoms associated with the infection). Exemplary animal models include a pregnant guinea pig model and mouse model (*e.g.,* see Busch et al., Animal model for infection with Listeria monocytogenes. Curr. Protoc. Immunol. 2001 May; Chapter 19:Unit 19.9) for *Listeria monocyotgenes;* mice and ferret models for influenza (*e.g.,* see Smee et al., Treatment of influenza A (H1N1) virus infections in mice and ferrets with cyanovirin-N. Antiviral Res. E-published 2008 Jul 2); and a mouse model for *Plasmodium falciparum* malaria (*e.g.,* see Angula-Barturen et al., PLoS ONE. 2008 May 21; 3(5):e2252). A candidate agent that decreases infection may be considered as an agent having antiviral potential.

### EXAMPLE 1

### Materials and Methods

This example describes the materials and methods use din Examples 2-5 below.

### Mouse bone marrow cultures

Bone marrow cells were isolated from tibias and femurs of 6-8 week old mice as described (22). Cells were differentiated for 7 days in RPMI containing 20 ng/ml mouse GM-CSF (Akron Biotech), 5% FBS (SAFC Biosciences) and antibiotic-antimycotic cocktail (Invitrogen) to generate bone marrow dendritic cells (BMDCs). Before stimulation, cells were starved overnight in serum-free RPMI. Bone marrow derived macrophages (BMDMs) were differentiated for 7 days in DMEM containing 10% FBS (SAFC Biosciences) and antibiotic-antimycotic cocktail (Invitrogen) and 100 ng/ml mM-CSF (PeproTech).

### Virus production and infections

Pseudotyped viruses were produced as described previously (23). Viruses were treated with 40 U/ml of DNAse I (Roche) for 1 hour to remove any residual plasmid DNA before adding to cells. MAV-1 was a generous gift from Colin Powers and Clodagh O' Shea (Salk Institute). BMDCs were aliquoted at 10⁵ cells/sample in eppendorf tubes and spinoculated with 500 ml of each pseudotyped virus at 1200 g for 1 hour followed by incubation at 37°C. HIV-1 DNA products were measured by qPCR analysis and those from knockout animals were normalized to cellular β-actin DNA levels before they were compared to the viral DNA level in wild-type BMDCs. The oligonucleotide primers (Invitrogen) and Taqman® probes (Applied Biosystems) used for the qPCR assays are listed in Table 1. For measuring HIV-1 DNA products, a standard curve was generated using six 10-fold serial dilutions of HIV-1 plasmid DNA. For measuring β-actin, a standard curve was generated using six 10-fold serial dilutions of uninfected BMDCs.

**Table 1. Oligonucleotide primers and probes**

| **Gene Name** | **Sequence (5 -> 3')** | **Citation** |
|---|---|---|
| **Taqman® Primers and Probes** | | |
| HIV Early RT Forward | GTGCCCGTCTGTTGTGTGAC (SEQ ID NO: 1) | Munk et al., PNAS, 99(21), 13843-48, 2002 |
| HIV Early RT Reverse | GGCGCCACTGCTAGAGATTT (SEQ ID NO: 2) | |
| HIV Early RT Probe | 6FAM-CTAGAGATCCCTCAGACCCTTTTAGTCAGTGTGG-TAMRA (SEQ ID NO: 3) | |
| PBGD Forward | AAGGGATTCACTCAGGCTCTTTC (SEQ ID NO: 4) | |
| PBGD Reverse | GGCATGTTCAAGCTCCTTGG (SEQ ID NO: 5) | |
| PBGD Probe | VIC-CCGGCAGATTGGAGAGAAAAGCCTGT-MGB (SEQ ID NO: 6) | |
| Mouse Beta-Actin Primer Probe Mix | Mouse ACTB (20X), Applied Biosystems Cat # 4352341E | |
| | | |

| **SYBR Green Primers** | | |
|---|---|---|
| IFN-beta Forward | ATGAGTGGTGGTTGCAGGC (SEQ ID NO: 7) | Rothlin et al., Cell, 131:1124-36,2007 |
| IFN-beta Reverse | TGACCTTTCAAATGCAGTAGATTCA (SEQ ID NO: 8) | |
| SOCS 1 Forward | CCGTGGGTCGCGAGAAC (SEQ ID NO: 9) | |
| SOCS 1 Reverse | AACTCAGGTAGTCACGGAGTACCG (SEQ ID NO: 10) | |
| SOCS3 Forward | TCCCATGCCGCTCACAG (SEQ ID NO: 11) | |
| SOCS3 Reverse | ACAGGACCAGTTCCAGGTAATTG (SEQ ID NO: 12) | |
| IRF7 Forward | ACAGGGCGTTTTATCTTGCG (SEQ ID NO: 13) | |
| IRF7 Reverse | TCCAAGCTCCCGGCTAAGT (SEQ ID NO: 14) | |
| Beta-Actin Forward | CTCCTCCTGAGCGCAAGTACTCTGTGT (SEQ ID NO: 15) | |
| Beta-Actin Reverse | GTGCACGATGGAGGGGCCGGACTCAT (SEQ ID NO: 16) | |
| IRF3 Forward | GAGAGCCGAACGAGGTTCAG (SEQ ID NO: 17) | |
| IRF3 Reverse | CTTCCAGGTTGACACGTCCG (SEQ ID NO: 18) | |
| IRF5 Forward | GGTCAACGGGGAAAAGAAACT (SEQ ID NO: 19) | |
| IRF5 Reverse | CATCCACCCCTTCAGTGTACT (SEQ ID NO: 20) | |
| IFN-alpha 4 Forward | TGATGAGCTACTACTGGTCAGC (SEQ ID NO: 21) | |
| IFN-alpha 4 Reverse | GATCTCTTAGCACAAGGATGGC (SEQ ID NO: 22) | |

BMDCs were pre-treated with 0 or 100 mg each of neutralizing anti-mouse interferon alpha and beta antibodies (PBL, IFNα Ab Cat. # 22100-1, IFNβ Ab Cat. # 22400-1) for 3 hours and then spinoculated with VSVg pseudotyped virus at 1200 g for 1 hour followed by incubation at 37°C either in the presence or absence of antibody. Reverse transcribed HIV-1 DNA products were measured at 24 hours post-infection as described above. For the BMS-77607 experiments, BMDCs obtained from wild-type or triple-TAM receptor knockout mice were incubated with the EbGP-pseudotyped virus either in the absence or presence of 300 nM BMS-777607 and the levels of HIV-1 DNA were measured at 24 hours post-infection.

### Immunoprecipitations and immunoblotting

HEK 293 cells stably overexpressing the TAM receptors were grown to 80% confluency in Dulbecco's Modified Eagle Medium supplemented with 10% fetal bovine serum, 100U/mL penicillin, and 100µg/mL streptomycin. Cells were washed in ice cold PBS and lysed in buffer containing 50mM Tris, pH7.5, 150mM NaCl, 1mM EDTA, 1mM EGTA, 10% glycerol, 1% triton X-100 supplemented with protease inhibitor cocktail (Roche) and phosphatase inhibitor cocktail (Roche). For immunopreciptations, cell lysates were incubated with antibodies directed against Tyro3 (Santa Cruz), Axl (Santa Cruz), and Mer (Prasad et al, 2006), respectively.

BMDCs were incubated with serum-free medium for 24 hours and then for 5 minutes with increasing amounts of either human Protein S (Haematologic Technologies Inc.) or mGas6, in the presence or absence of VSVg pseudotyped virus or MAV-1. The cells were washed once with cold PBS and then lysed on ice in a lysis buffer containing 50 mM Tris-HCl pH 7.5, 1 mM EGTA, 1 mM EDTA, 1% (w/v) Triton X-100, 0.27 M sucrose, 0.1% beta-mercaptoethanol, protease inhibitor cocktail (Roche) and phosphatase inhibitor cocktail (Roche). Protein concentration was measured using Bradford reagent (Bio-Rad).

For western blotting equal amounts of protein (5 µg) in LDS sample buffer (Invitrogen) were subjected to electrophoresis on a polyacrylamide gel and transferred to PVDF membranes (Millipore). For immunoprecipitation cell lysates (0.1 mg protein) were incubated with 0.2 µg antibody overnight at 4°C on a rotating wheel: anti-Mer (in-house rabbit polyclonal (*18*); anti-Axl (goat polyclonal, M-20, Santa Cruz); anti-Tyro3 (goat polyclonal, C-20, Santa Cruz); anti-GAPDH (mouse monoclonal, clone 6C5, Millipore); and Anti-pTyr (mouse monoclonal, clone 4G10, Millipore). Protein-G/A Sepharose (Invitrogen) was added for additional 2 h and immunoprecipitates were washed twice with 1 ml of Lysis Buffer containing 0.5 M NaCl and once with 1 ml of 50 mM Tris/HCl pH 7.5. Immunoprecipitates were eluted in LDS Sample Buffer, separated on polyacrylamide gels and transferred to PVDF membranes. Membranes were blocked in TBS-T (50 mM Tris-HCl pH 7.5, 0.15M NaCl, and 0.25% (v/v) Tween-20) containing in 5% (w/v) BSA and then immunoblotted overnight at 4°C with primary antibodies diluted 1000-fold in blocking buffer. The antibodies used were the same as above except that a different anti-Mer antibody (goat polyclonal, AF591, R&D), was used. The blots were washed six times with TBS-T and incubated for 1 hour at room temperature with secondary HRP-conjugated antibodies (GE Healthcare) diluted 5000-fold in 5% (w/v) skimmed milk in TBS-T. After repeating the wash steps, the signal was detected with the enhanced chemiluminescence reagent and immunoblots were developed using an automatic film processor.

Endogenous TAM receptor expression was induced on wild-type BMDMs with 0.1 µM dexamethasone (24 hours) for Mer, 10 µg/ml poly(I:C) (InvivoGen) for Axl, or 10 µM TGF-βR Inhibitor, SB431542 (Selleck Chemicals) (7 days), for Tyro3. Cells were starved overnight and then pre-treated for 30 min with BMS-777607 (Selleck Chemicals) compound or equivalent volume of DMSO (final DMSO concentration was less than 0.01%). Cells were then stimulated with 10 nM Gas6 for 10 minutes. Immunopreciptation and immunoblot analysis was conducted as described above.

### Quantitative RT-PCR analysis

BMDCs were infected by spinoculation with each pseudotyped virus at 1200 g for 1 hour followed by incubation at 37°C for time points up to 24 h. RNA was isolated from the cells with the RNeasy mini kit (Qiagen) and cDNA was prepared with the QuantiTect reverse transcription kit (Qiagen). qPCR reactions were performed to measure mRNA expression levels with the oligonucleotide primers listed in Table 1, using Power SYBRGreen PCR master mix (Applied Biosystems). A dissociation curve analysis was performed for each reaction with the SDS software (Applied Biosystems). A standard curve was generated using six 10-fold dilutions of samples from uninfected BMDCs.

### Purification of recombinant mouse Gas6

A mammalian expression vector was engineered to encode full-length mouse Gas6 followed by a C-terminal, TEV-cleavable His₆-tag. The construct was transfected into 293T cells, and cells stably expressing the construct were selected in Dulbecco's Modified Eagle Medium supplemented with 10% fetal bovine serum, 100U/mL penicillin, 100g/mL streptomycin 0.25mg/mL G418, and 100µg/mL hygromycin B. For expression studies, cells were grown in serum free medium supplemented with 10µM Vitamin K2, and conditioned medium was collected after 72 hours. Secreted Gas6 was isolated using affinity chromatography with Ni-NTA beads followed by additional purification on a Hi Trap Q Fast Flow ion exchange column (GE Healthcare). The protein was eluted in 20mM Tris, pH 8 with a sodium chloride gradient up to 1M.

### Example 2

### Impact of Specific TAM Receptors on Virus Infection

Loss-of-function studies were performed with bone marrow-derived dendritic cells (BMDCs) prepared from either wild type (WT) or specific TAM gene knockout mice (*17*), which were challenged with HIV-1-derived viruses pseudotyped with one of four different viral glycoproteins - Ebola virus GP, Marburg virus GP, VSVg, and amphotropic MLV Env. Wild-type BMDCs (WT-BMDCs) and macrophages express Mer and Axl, but only very low levels of Tyro3 (5).

As shown in FIG. 1B, efficient cellular entry by all four HIV-1-derived pseudotypes was critically dependent upon BMDC expression of endogenous TAM receptors. The experiments were conducted with serum-supplemented cell culture medium that contained Protein S, a Mer and Tyro3 ligand (*2, 3, 18*), but not Gas6, a pan-TAM ligand (*2, 3*). [Serum contains only very low levels of Gas6 (~0.2nM), all of which is complexed with soluble Axl ectodomain (*19*)]. Consistent with its expression pattern and ligand-specificity, efficient pseudotyped virus infection of BMDCs was mostly dependent upon Mer expression; since the low levels of viral entry seen with TAM triple knockout (TKO) animals were recapitulated with cells derived from either Mer single knockout or Axl/Mer double knockout mice (FIG. 1B). Compared to Mer and Tyro3 KOs, loss of Axl, which in other cell types has been suggested to be a filovirus-specific entry cofactor (*8, 9, 12, 13*) had little effect on the efficiency of BMDC infection by the viral pseudotypes (FIG. 1B). This is consistent with the fact that the Axl ligand Gas6 was not present in the experiment.

### Example 3

### Enveloped Virus Potentiates Ligand-Dependent TAM Receptor Activation During Virus Entry

This example describes results showing that the TAM RTKs are directly activated by ligand-coated virus particles during cellular entry and that this activation in turn inhibits innate immune responses that would otherwise antagonize virus infection.

To determine if endogenous TAM receptors are activated upon virus challenge, WT-BMDCs were challenged with a VSVg pseudotyped virus either in the presence or absence of purified Protein S or Gas6 (see Example 1). For these experiments, the cell culture medium used during virus production was first depleted of all Gla-domain containing proteins, including Protein S, using sodium citrate and barium chloride precipitation (*20*). Addition of Protein S or Gas6 alone to cells led to a rapid dose-dependent activation of Mer or Axl, respectively (FIG. 2A, B left panels). However, addition of the VSVg pseudotyped virus dramatically potentiated the stimulatory effects of both Gas6 and Protein S, shifting the dose-response curves of these TAM ligands to lower protein concentrations (FIG. 2A, B left panels).

Importantly, virus-mediated potentiation of TAM ligand signaling was not seen with mouse adenovirus type 1 (MAV-1), a control non-enveloped virus that does not display PtdSer and therefore should not bind either TAM ligand (FIG. 2A, B right panels). These data indicate that molecules of Protein S or Gas6, displayed on the enveloped virus surface, act as virion-tethered "super-ligands" that immediately and strongly activate endogenous TAM receptors during the earliest steps of virus entry.

### Example 4

### Enveloped Virus Infection Abrogates the Cellular Antiviral Response in a TAM-Dependent Manner

TAM receptor activation in DCs leads to up-regulated expression of the Suppressors of Cytokine Signaling proteins (SOCS)1 and SOCS3, and consequently to inhibition of both expression of and signaling by host cytokines, including type I interferons (IFNs) (*5*). Since the latter are potent anti-viral agents, it was determined whether virus-mediated activation of TAM receptors blunts the cellular antiviral response.

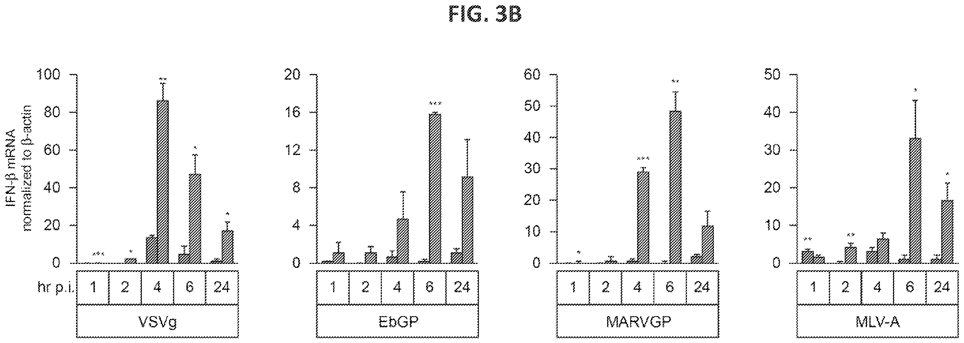

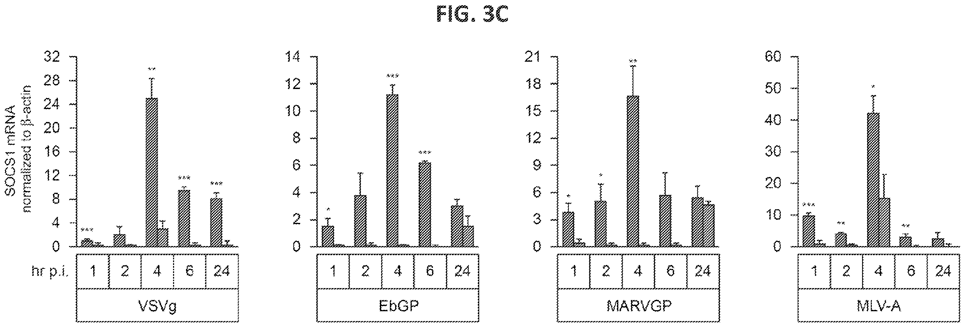

BMDCs derived from WT or *Tyro3*^{*-*/}*⁻Axl*^{*-*/}*⁻Mer*^{*-*/*-*} (TAM TKO) mice (*5, 7, 17*) were challenged with the four pseudotyped viruses, and the expression of innate immune modulator genes was monitored at multiple time points (from 1-24 hours) following virus addition. Virus challenge resulted in a minimal induction of mRNAs encoding type I IFNs (IFNβ and IFNα4) in WT-BMDCs (FIGS. 3A, B). In marked contrast, the same challenge led to a dramatic induction of these cytokine mRNAs (ranging from 20-80 fold) in TAM TKO BMDCs (FIGS. 3A, B). Virus challenge also led to a significant elevation of tumor necrosis factor (TNF)α, interferon regulatory factor (IRF)-3, IRF-5, and IRF-7 mRNAs in TAM TKO BMDCs, but not in WT-BMDCs (FIGS. 3D-3H). Importantly, the reciprocal effect was seen for SOCS1 and SOCS3 mRNA: these mRNAs were markedly up-regulated subsequent to virus infection in WT-BMDCs, with almost no up-regulation in response to virus in TAM TKO BMDCs (FIGS. 3C and 3D-3H).

Together, these results demonstrate that TAM receptor activation during enveloped virus infection of DCs effectively shuts down the cellular innate immune response to infection.

### Example 5

### Effect of TAM Receptor-Ligand Interactions on Virus Infection in BMDCs is Through Inhibition of the Cellular Antiviral Response

To determine the specific contribution of this mechanism to the overall efficiency of enveloped virus infection of TAM expressing cells, neutralizing antibodies that bind to interferon α and β were used to block the action of type I IFNs during virus challenge of BMDCs.

The antibody cocktail had no impact on the level of infection seen with WT-BMDCs (FIG. 4A), an expected result since interferon is not induced in these cells following virus challenge (FIGS. 3A, B). Strikingly however, these antibodies significantly restored the level of infection seen with TAM-TKO BMDCs to a level similar to that seen with WT-BMDCs (FIG. 4A). Therefore, the major enhancing effect of virus-mediated TAM activation during dendritic cell infection is through inhibition of the host antiviral response.

These data indicate that antagonists targeting TAM receptors, such as small molecule TAM-restricted tyrosine kinase inhibitors, can have a novel antiviral effect through their ability to block virus-induced TAM-dependent attenuation of type I IFN signaling. To demonstrate this, BMS-777607, an ATP mimetic that targets the closely related c-Met/TAM/Ron tyrosine kinases that has been used in phase I and phase II clinical trials for the treatment of advanced or metastatic solid tumors (*21*) was used. This drug is a potent inhibitor of the ligand-dependent activation of both Axl and Mer in cultured cells, effectively inhibiting receptor activation at concentrations ranging from 30-300 nM (FIG. 4B). As compared with untreated WT BMDCs, those treated with BMS-777607 were much less susceptible to virus infection (FIG. 4C). Importantly, the infection deficiency seen with TAM TKO BMDCs was not further reduced by the addition of BMS-777607, providing direct evidence that the drug reduces virus infectivity in wild-type cells by blocking the activation of TAM receptors as opposed to other receptor tyrosine kinases.

### Example 6

### A PtdSer-containing membrane envelope is necessary for virus activation of TAM signaling.

This example shows results that demonstrate that PtdSer exposed on membranes is critical for viral infectivity and increase of agonist activity of Gas6 and Protein S.

Virus-mediated potentiation of Protein S- or Gas6-triggered TAM ligand signaling was not seen with mouse adenovirus type 1 (MAV-1), a control non-enveloped virus that does not display PtdSer and therefore should not bind either TAM ligand. Correspondingly, MAV-1 challenge of WT and AM DKO BMDCs resulted in comparable induction of IFNα4, IFNβ, TNFα, and SOCS1/3 mRNAs; and the efficiency of MAV-1 infection in BMDCs was not influenced by the presence or absence of TAM receptors (FIG. 5A). MAV-1 was grown on mouse 3T6 cells, concentrated from supernatant using PEG precipitation, and purified on a CsCl gradient. Purified virus was dialyzed in 10% glycerol, 10mM Tris pH7.5, 150mM NaCl, and 1mM MgCl₂, and titered by plaque assay on 3T6 cells. BMDCs were infected with MAV-1 at MOI of 1. Cells were lysed 24 h postinfection, and expression of early adenovirus gene E1A was assayed by qPCR and normalized to endogenous Cyclophilin A.

These results indicate that virus-associated PtdSer is required for TAM receptor activation. To confirm, the ability of Annexin V, a known PtdSer-binding protein, to inhibit the effect of membranes on Gas6 and Protein S was determined. Incubation of VSVg-pseudotyped virus with a limiting concentration (100 nM) of the PtdSer-binding protein Annexin V significantly attenuated VSVg-HIV potentiation of the Gas6 dose-response curve for Axl activation in BMDCs (FIG. 5B). Experiments were performed and Axl activation (autophosphorylation) assayed as described for FIG. 2, but in the absence (-) or presence (+) of 100 nM of the PtdSer-binding protein Annexin V. This concentration of Annexin V is approximately 7-fold lower than that used previously by Meertens et al. (2012) to achieve a ∼60% reduction in DENV infection of Axl-expressing HEK293 cells.

### Example 7

### Retrovirus potentiation of TAM signaling is independent of any viral glycoprotein.

This example shows results that demonstrate that binding of Gas6 or Protein S to phosphatidylserine-containing membranes, rather than any viral factors that might be present on the extracellular surface of the membrane, is critical for the increased agonist activity.

To assess the impact of viral factors, "bald" HIV-1 virions lacking any viral glycoproteins were generated. Bald virions were non-infectious in BMDCs (FIG. 6A). However, they were equally competent as VSVg-pseudotyped virions at potentiating Gas6-induced Axl activation (FIG. 6B). Thus, the ability of the pseudotyped virus to activate TAM receptors was dependent on the presence of PtdSer but independent of any specific viral glycoprotein, demonstrating that the change of ligand properties is mediated by PtdSer-containing membranes, not viral glycoproteins or other viral factors. This change in ligand properties towards TAM receptors, induced by an interaction between lipids and the gla-domain, provides a novel approach to modulate ligand-receptor interaction and function.

### References

1. C. Lai, G. Lemke, Neuron 6, 691 (May, 1991).
2. G. Lemke, C. V. Rothlin, Nat Rev Immunol 8, 327 (May, 2008).
3. T. N. Stitt et al., Cell 80, 661 (Feb 24, 1995).
4. G. Lemke, T. Burstyn-Cohen, Ann N Y Acad Sci 1209, 23 (Oct, 2010).
5. C. V. Rothlin, S. Ghosh, E. I. Zuniga, M. B. Oldstone, G. Lemke, Cell 131, 1124 (Dec 14, 2007).
6. G. Lemke, Q. Lu, Curr Opin Immunol 15, 31 (Feb, 2003).
7. Q. Lu, G. Lemke, Science 293, 306 (2001).
8. C. L. Hunt, A. A. Kolokoltsov, R. A. Davey, W. Maury, J Virol 85, 334 (Jan, 2011).
9. M. A. Brindley et al., Virology 415, 83 (Jul 5, 2011).
10. M. Shimojima, U. Stroher, H. Ebihara, H. Feldmann, Y. Kawaoka, J Virol, (Dec 7, 2011).
11. K. Morizono et al., Cell Host Microbe 9, 286 (Apr 21, 2011).
12. M. Shimojima, Y. Ikeda, Y. Kawaoka, J Infect Dis 196 Suppl 2, S259 (Nov 15, 2007).
13. M. Shimojima et al., J Virol 80, 10109 (Oct, 2006).
14. J. Mercer, Cell Host Microbe 9, 255 (Apr 21, 2011).
15. T. Burstyn-Cohen, M. J. Heeb, G. Lemke, J Clin Invest 119, 2942 (Oct, 2009).
16. P. Garcia de Frutos, P. Fuentes-Prior, B. Hurtado, N. Sala, Thromb Haemost 98, 543 (Sep, 2007).
17. Q. Lu et al., Nature 398, 723 (Apr 22, 1999).
18. D. Prasad et al., Mol Cell Neurosci 33, 96 (Sep, 2006).
19. C. Ekman, J. Stenhoff, B. Dahlback, J Thromb Haemost 8, 838 (Apr, 2010).
20. M. Souri, S. Koseki-Kuno, H. Iwata, B. Kemkes-Matthes, A. Ichinose, Blood 105, 3149 (Apr 15, 2005).
21. G. M. Schroeder et al., J Med Chem 52, 1251 (Mar 12, 2009).
22. C. V. Harding, D. Canaday, L. Ramachandra, Curr Protoc Immunol Chapter 16, Unit 16 1 (Feb, 2010).
23. S. Bhattacharyya, T. J. Hope, J. A. Young, Virology 419, 1 (Oct 10, 2011).

In view of the many possible embodiments to which the principles of the disclosure may be applied, it should be recognized that the illustrated embodiments are only examples of the disclosure and should not be taken as limiting the scope of the invention. Rather, the scope of the disclosure is defined by the following claims. We therefore claim as our invention all that comes within the scope and spirit of these claims.
The application discloses inter alia the following items
1. A method of treating an autoimmune disease in a subject, comprising:
   administering to the subject a therapeutically effective amount of a TAM receptor agonist comprising a phosphatidyl serine (PtdSer) -containing lipid bilayer membrane with Gas6 and/or Protein S bound to the membrane, thereby activating signaling from one or more TAM receptors, thereby treating the autoimmune disease.
2. The method of item 1, wherein the autoimmune disease is selected from the group consisting of amyotriphic lateral sclerosis, celiac disese, rheumatoid arthritis, Hashimoto's thyroiditis, pernicious anemia, inflammatory bowel disease (Crohn's disease and ulcerative colitis), Cushing's syndrome, Guillain-Barre syndrome, transverse myelitis, psoriasis, renal, pulmonary, and hepatic fibroses, Addison's disease, type I diabetes, systemic lupus erythematosus, dermatomyositis, Sjogren's syndrome, multiple sclerosis, myasthenia gravis, Reiter's syndrome, and Grave's disease.
3. The method of any ofitems 1-2, wherein the membrane is a liposome or a nanoparticle.
4. The method of any of items 1-3, wherein the subject is a human.
5. The method of any of items 1-4, wherein the TAM receptor is Axl, Mer and/or Tyro3.
6. The method of any of items 1-5, wherein Gas6 and/or Protein S binds to the lipid membrane through the PtsdSer-Gla domain interaction.
7. A composition comprising a PtdSer-containing membrane having a Gas6 and/or Protein S protein bound to said membrane.
8. The composition of item 7, wherein the PtdSer containing membrane comprises a liposome or a nanoparticle.
9. A method for classifying a virus as susceptible to anti-TAM therapy comprising:
   i) determining whether the virus comprises PtdSer on the envelope surface; and/or
   ii) determining whether the virus induces TAM signaling when in contact with a TAM ligand; and
   iii) comparing a value for i) and/or ii) with a value for a virus with a known amount of PtdSer on the envelope surface and/or a virus known to induce TAM signaling, thereby classifying the virus as susceptible or resistant to anti-TAM therapy.
10. The method of item 9, wherein the anti-TAM therapy is a small molecule inhibitor of TAM intracellular signaling.
11. A method of identifying an agent that blocks virus infectivity comprising:
   contacting one or more test agents with a virus and an immune cell; and
   determining TAM receptor activation in the presence and absence of the test agent(s),
   wherein decreased TAM receptor activation in the presence of the test agent is indicative of an agent that blocks virus infectivity.
12. A method of treating a subject with a pathological condition characterized by overactivation of TAM signaling and/or reduction in Type I IFN response with an agent disrupting the interaction between Gas6 and/or Protein S with PtdSer-containing lipid bilayer membranes.
13. The method of item 12, wherein the pathological condition is a virus infection by an enveloped virus.
14. The method of item 12 or 13, wherein the agent disrupting the interaction between Gas6 and/or Protein S with PtdSer is an antibody to the Gla-domain of Gas6 and/or Protein S.
15. The method of item 12 or 13, wherein the agent disrupting the interaction between Gas6 and/or Protein S with PtdSer is a full length Gla-domain derived from human Gas6 or Protein S sequence, or a fragment thereof.
16. The method of item 12 or 13, wherein the agent disrupting the interaction between Gas6 and/or Protein S with PtdSer is an antibody to PtdSer.
17. The method of item 12 or 13, wherein the agent disrupting the interaction between Gas6 and/or Protein S with PtdSer is a natural or artificial peptide binding PtdSer.

## Claims

1. TAM receptor agonist for use in a method of treating an autoimmune disease in a subject, wherein the TAM receptor agonist comprises a phosphatidyl serine (PtdSer) - containing lipid bilayer membrane with Gas6 and/or Protein S bound to the membrane, wherein signaling from one or more TAM receptors is activated.

2. The TAM receptor agonist for use of claim 1, wherein the autoimmune disease is selected from the group consisting of amyotriphic lateral sclerosis, celiac disese, rheumatoid arthritis, Hashimoto's thyroiditis, pernicious anemia, inflammatory bowel disease (Crohn's disease and ulcerative colitis), Cushing's syndrome, Guillain-Barre syndrome, transverse myelitis, psoriasis, renal, pulmonary, and hepatic fibroses, Addison's disease, type I diabetes, systemic lupus erythematosus, dermatomyositis, Sjogren's syndrome, multiple sclerosis, myasthenia gravis, Reiter's syndrome, and Grave's disease.

3. The TAM receptor agonist for use of claim 1 or 2, wherein the membrane is a liposome or a nanoparticle.

4. The TAM receptor agonist for use of any one of claims 1 to 3, wherein the subject is a human.

5. The TAM receptor agonist for use of any one of claims 1 to 4, wherein the TAM receptor is Axl, Mer and/or Tyro3.

6. The TAM receptor agonist for use of any of claims 1 to 5, wherein Gas6 and/or Protein S binds to the lipid membrane through the PtsdSer-Gla domain interaction.

7. A composition comprising a PtdSer-containing membrane having a Gas6 and/or Protein S protein bound to said membrane, preferably wherein the PtdSer containing membrane comprises a liposome or a nanoparticle.

8. A method for classifying a virus as susceptible to anti-TAM therapy comprising:
i) determining whether the virus comprises PtdSer on the envelope surface; and/or
ii) determining whether the virus induces TAM signaling when in contact with a TAM ligand; and
iii) comparing a value for i) and/or ii) with a value for a virus with a known amount of PtdSer on the envelope surface and/or a virus known to induce TAM signaling,
thereby classifying the virus as susceptible or resistant to anti-TAM therapy, optionally wherein the anti-TAM therapy is a small molecule inhibitor of TAM intracellular signaling.

9. A method of identifying an agent that blocks virus infectivity comprising:
i) contacting one or more test agents with a virus and an immune cell; and
ii) determining TAM receptor activation in the presence and absence of the test agent(s),
wherein decreased TAM receptor activation in the presence of the test agent is indicative of an agent that blocks virus infectivity.

10. A agent for use in a method of treating a subject with a pathological condition **characterized by** overactivation of TAM signaling and/or reduction in Type I IFN response, wherein the agent disrupts the interaction between Gas6 and/or Protein S with PtdSer-containing lipid bilayer membranes.

11. The agent for use of claim 10, wherein the pathological condition is a virus infection by an enveloped virus.

12. The agent for use of claim 10 or 11, wherein the agent disrupting the interaction between Gas6 and/or Protein S with PtdSer is an antibody to the Gla-domain of Gas6 and/or Protein S.

13. The method of claim 10 or 11, wherein the agent disrupting the interaction between Gas6 and/or Protein S with PtdSer is a full length Gla-domain derived from human Gas6 or Protein S sequence, or a fragment thereof.

14. The agent for use of claim 10 or 11, wherein the agent disrupting the interaction between Gas6 and/or Protein S with PtdSer is an antibody to PtdSer.

15. The agent for use of claim 10 or 11, wherein the agent disrupting the interaction between Gas6 and/or Protein S with PtdSer is a natural or artificial peptide binding PtdSer.


SEQUENCE LISTING

<110>  Salk Institute for Biological Studies
       Xetrios Therapeutics, Inc.

<120>  REGULATING THE INTERACTION BETWEEN TAM LIGANDS AND LIPID
       MEMBRANES WITH EXPOSED PHOSPHATIDYLSERINE

<130>  7158-89259-02

<150>  61/675,731
<151>  2012-07-25

<160>  22

<170>  PatentIn version 3.5

<210>  1
<211>  20
<212>  DNA
<213>  Artificial Sequence

<220>
<223>  HIV Early RT Forward primer

<400>  1
gtgcccgtct gttgtgtgac                                                   20


<210>  2
<211>  20
<212>  DNA
<213>  Artificial Sequence

<220>
<223>  HIV Early RT Reverse primer

<400>  2
ggcgccactg ctagagattt                                                   20


<210>  3
<211>  34
<212>  DNA
<213>  Artificial Sequence

<220>
<223>  HIV Early RT Probe

<400>  3
ctagagatcc ctcagaccct tttagtcagt gtgg                                   34


<210>  4
<211>  23
<212>  DNA
<213>  Artificial Sequence

<220>
<223>  PBGD Forward primer

<400>  4
aagggattca ctcaggctct ttc                                               23

<210>  5
<211>  20
<212>  DNA
<213>  Artificial Sequence

<220>
<223>  PBGD Reverse primer

<400>  5
ggcatgttca agctccttgg                                                    20


<210>  6
<211>  26
<212>  DNA
<213>  Artificial Sequence

<220>
<223>  PBGD Probe

<400>  6
ccggcagatt ggagagaaaa gcctgt                                             26


<210>  7
<211>  19
<212>  DNA
<213>  Artificial Sequence

<220>
<223>  IFN-beta Forward primer

<400>  7
atgagtggtg gttgcaggc                                                     19


<210>  8
<211>  25
<212>  DNA
<213>  Artificial Sequence

<220>
<223>  IFN-beta Reverse primer

<400>  8
tgacctttca aatgcagtag attca                                              25


<210>  9
<211>  17
<212>  DNA
<213>  Artificial Sequence

<220>
<223>  SOCS1 Forward primer

<400>  9
ccgtgggtcg cgagaac                                                       17


<210>  10

<211>  24
<212>  DNA
<213>  Artificial Sequence

<220>
<223>  SOCS1 Reverse primer

<400>  10
aactcaggta gtcacggagt accg                                              24


<210>  11
<211>  17
<212>  DNA
<213>  Artificial Sequence

<220>
<223>  SOCS3 Forward primer

<400>  11
tcccatgccg ctcacag                                                      17


<210>  12
<211>  23
<212>  DNA
<213>  Artificial Sequence

<220>
<223>  SOCS3 Reverse primer

<400>  12
acaggaccag ttccaggtaa ttg                                               23


<210>  13
<211>  20
<212>  DNA
<213>  Artificial Sequence

<220>
<223>  IRF7 Forward primer

<400>  13
acagggcgtt ttatcttgcg                                                   20


<210>  14
<211>  19
<212>  DNA
<213>  Artificial Sequence

<220>
<223>  IRF7 Reverse primer

<400>  14
tccaagctcc cggctaagt                                                    19


<210>  15
<211>  27
<212>  DNA
<213>  Artificial Sequence

<220>
<223>  Beta-Actin Forward primer

<400>  15
ctcctcctga gcgcaagtac tctgtgt                                          27


<210>  16
<211>  26
<212>  DNA
<213>  Artificial Sequence

<220>
<223>  Beta-Actin Reverse primer

<400>  16
gtgcacgatg gaggggccgg actcat                                           26


<210>  17
<211>  20
<212>  DNA
<213>  Artificial Sequence

<220>
<223>  IRF3 Forward primer

<400>  17
gagagccgaa cgaggttcag                                                  20


<210>  18
<211>  20
<212>  DNA
<213>  Artificial Sequence

<220>
<223>  IRF3 Reverse primer

<400>  18
cttccaggtt gacacgtccg                                                  20


<210>  19
<211>  21
<212>  DNA
<213>  Artificial Sequence

<220>
<223>  IRF5 Forward primer

<400>  19
ggtcaacggg gaaaagaaac t                                                21


<210>  20
<211>  21
<212>  DNA
<213>  Artificial Sequence

<220>
<223>  IRF5 Reverse primer

<400>  20
catccacccc ttcagtgtac t                                                21

<210>  21
<211>  22
<212>  DNA
<213>  Artificial Sequence

<220>
<223>  IFN-alpha 4 Forward primer

<400>  21
tgatgagcta ctactggtca gc                                               22

<210>  22
<211>  22
<212>  DNA
<213>  Artificial Sequence

<220>
<223>  IFN-alpha 4 Reverse primer

<400>  22
gatctcttag cacaaggatg gc                                               22